# EUROPEAN PATENT SPECIFICATION

(11) **EP 0 422 175 B1**
(45) Date of publication and mention of the grant of the patent: **31.07.1996**
(21) Application number: 90906218.4
(22) Date of filing: 18.04.1990
(51) Int. Cl.: C07K 14/00, C12N 15/12, C12P 21/02, A61K 38/00

(54) **PROTEINS WHICH REGULATE THE EXPRESSION OF VERTEBRATE MHC CLASS II GENES, DNA SEQUENCES ENCODING THEM AND PHARMACEUTICAL COMPOSITIONS**
PROTEINE, DIE DIE EXPRESSION VON VERTEBRATEN-MHC-KLASSE-II-GENEN REGULIEREN, DAFÜR KODIERENDE DNA-SEQUENZEN UND PHARMAZEUTISCHE ZUSAMMENSETZUNGEN
PROTEINES REGLANT L'EXPRESSION DE GENES MHC CLASSE II DE VERTEBRES, SEQUENCES D'ADN LES ENCODANT ET COMPOSITIONS PHARMACEUTIQUES

(30) Priority: 18.04.1989 EP 89106944; 14.08.1989 EP 89115008
(43) Date of publication of application: 17.04.1991
(73) Proprietor: Mach, Bernard François, Prof., CH-1292 Chambesy/GE (CH)
(72) Inventor: Mach, Bernard François, Prof., CH-1292 Chambesy/GE (CH)
(74) Representative: VOSSIUS & PARTNER
(86) International application number: EP9000625
(87) International publication number: WO9012812

(56) References cited:
- EXPERIENTIA, vol. 45, February 1989, Basel (CH), Abstracts of the 21st Annual Meeting of the Swiss Societies for Experimental Biology, Fribourg (CH), 30-31 March 1989; W. REITH et al., p. A-22, AN 73
- PROCEEDINGS OF THE NATL. ACADEMY OF SCIENCES USA, vol. 85, October 1988, Washington, DC (US); D.K. Didier et al., pp. 7322-7326
- PROCEEDINGS OF THE NATL. ACADEMY OF SCIENCES USA, vol. 86, no. 11, June 1989, Washington, DC (US); W. REITH et al., pp. 4200-4204

## Description

The invention relates to proteins which regulate the expression of vertebrate MHC class II genes and bind to the X box of DNA sequences controlling the expression of said genes. Furthermore, the invention relates to DNA molecules encoding said proteins and corresponding recombinant DNA molecules, transformed microorganisms and their use for the production of said proteins. The invention additionally relates to pharmaceutical compositions for the treatment of diseases which are caused by an aberrant expression of MHC class II genes.

Class II major histocompatibility (MHC) antigens are heterodimeric transmembrane glycoproteins. Their expression at the surface of antigen presenting cells is essential for the recognition of foreign antigen by the T cell receptor. T cell activation and antigen presentation depend on the level of expression of class II antigens on individual cells. Regulation of expression of class II genes is therefore an important aspect of the control of both normal and abnormal immune responses.

In man, the genes encoding the a and b chains of the HLA-DP, HLA-DQ and HLA-DR class II molecules are clustered in the D region of the MHC on chromosome 6. These genes are subjected to tight and complex regulatory controls. Their expression is generally coordinated, and restricted primarily to cells of the immune system such as B lymphocytes, activated T lymphocytes, macrophages, dendritic cells, and certain specialized cells such as Kupfer cells and Langerhans cells. In certain class II negative cells, expression can be induced by stimulation with lymphokines such as interferon y or interleukin 4.

Several autoimmune diseases, such as Insulin dependant Diabetes, Multiple sclerosis, Rheumatoid arthritis, Lupus erythematosis, are thought to be due, at least in part, to aberrant expression of HLA class II antigens on cells that normally should not express them. Abnormal T cell activation then leads to the autoimmune process. Treatment of animals suffering from autoimmune diseases with antibodies directed against MHC class II molecules have already given encouraging results. It would consequently be very desirable to be able to down-regulate the expression of HLA class II genes in order to prevent or treat autoimmune diseases, for instance by acting on a possible switch mechanism which controls class II gene expression.

The exact mechanisms of class II gene regulation are not yet clearly understood. It is well established that DNA sequences upstream of class II genes are involved in this process. A number of factors, not fully characterized biochemically, can bind to this region of DNA and may thus be functionally relevant. It was observed that in cells from regulatory mutants defective in HLA class II gene expression (1), the pattern of binding to DNA is different, suggesting a defect in the binding of a given factor to DNA (2). Since in these mutant cells the expression of all class II genes is affected, such a defect in DNA binding probably affects the expression of all class II genes. This factor could not be isolated, purified or characterized and its exact biochemical nature could not be determined. Its binding site on DNA is the X box of class II promoters and consequently this factor was named RF-X (2). However, the chemical nature of the RF-X factor was not known. If this DNA-binding factor defective in the regulatory mutants were a protein, it could represent an essential regulator of class II gene expression. In such a case, access to such a protein, or to related proteins having substantially the same activity, and/or to their genes, could provide ways of modulating the regulation of HLA class II gene expression, a truly desirable objective for the treatment of important autoimmune diseases, as mentioned above.

Thus the technical problem underlying the present invention is to isolate and identify the RF-X factor and to provide it in purified form. Furthermore, the technical problem underlying the present invention is to provide proteins with the biological properties of the RF-X factor, DNA sequences encoding said RF-X factor and/or related proteins, recombinant DNA molecules containing these DNA sequences and microorganisms transformed with said recombinant DNA molecules. Still further, the technical problem is to provide recombinant DNA processes for the production of said proteins in which the transformed microorganisms are used. In addition, it is the technical problem to provide pharmaceutical compositions containing said proteins or corresponding DNA sequences.

The above technical problem is solved by providing the embodiments of the present invention which are characterized in the claims.

Accordingly, proteins are provided which normally occur in vertebrate cells expressing MHC class II genes, regulate the expression of vertebrate MHC class II genes, and bind to the X box of DNA sequences controlling the expression of said MHC class II genes.

A particular obstacle which had to be overcome in order to provide said proteins was that the still unidentified and impure factor RF-X described in (2), in contrast to other DNA-binding factors, does not bind to short DNA segments of 20 to 25 base pairs. As a consequence, with the use of short DNA segments (of 20 to 25 base pairs) normally employed as target sequence in such binding experiments, a gene encoding a protein with the properties of factor RF-X could not be identified or isolated. During a short oral presentation made by B. Mach at the 21st USGEB meeting in March 1989, the importance of HLA class II genes in the control of immune response was discussed. Furthermore, in this presentation it was reported that a gene encoding a X box binding protein was isolated, however no details with respect to the complex strategy of isolating the gene were given.

Another obstacle which had to be overtaken in order to provide said protein is the failure of several MHC class II genes, such as HLA-DQA in humans, or IE α in mice, to bind the RF-X factor efficiently. Therefore, utilising HLA-DRA rather than some of the other MHC class II genes as mentioned above was an essential step to the successful identification of RF-X and of its gene.

The term "regulation" means that the purified proteins of the present invention control the expression of vertebrate MHC class II genes. The term "binding" means that said proteins interact with the X box of the DNA sequence that control the expression of said MHC class II genes.

It can be started from the assumption that the proteins of the present invention naturally occur in all vertebrates, because all vertebrates have MHC class II-like molecules on the surface of a particular cell compartment. Examples are B lymphocytes or macrophages of mice or chicken.

In a preferred embodiment of the present invention, the purified proteins are those which naturally occur in mammalians, preferably in humans.

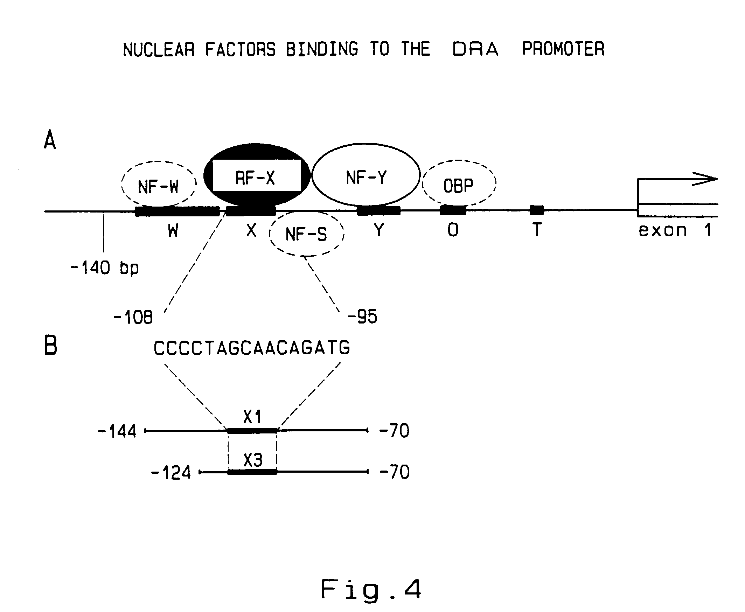

A further preferred embodiment are proteins exhibiting the abovementioned properties which have the biological activity of the RF-X protein which naturally occurs in human cells expressing MHC class II genes and which bind to oligonucleotides containing the sequences -70 to -144 (X1) or -70 to -124 (X3) of the DRA promoter shown in Figure 4. In this context, the term "having the biological activity of the RF-X protein" means proteins that are capable of binding to the controlling region ("promoter") of the MHC class II genes or proteins which are essential for the expression of MHC class II genes.

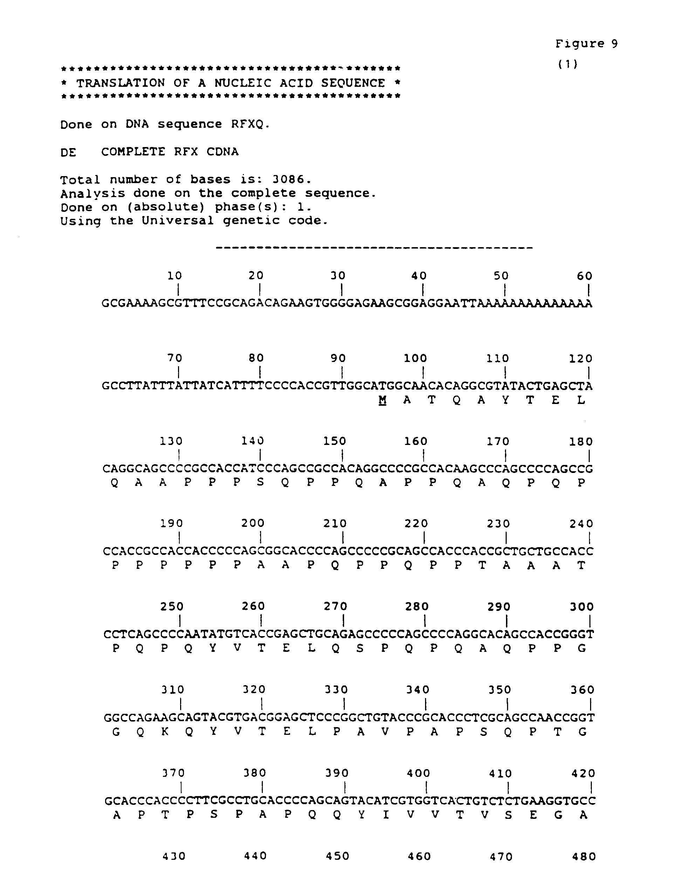

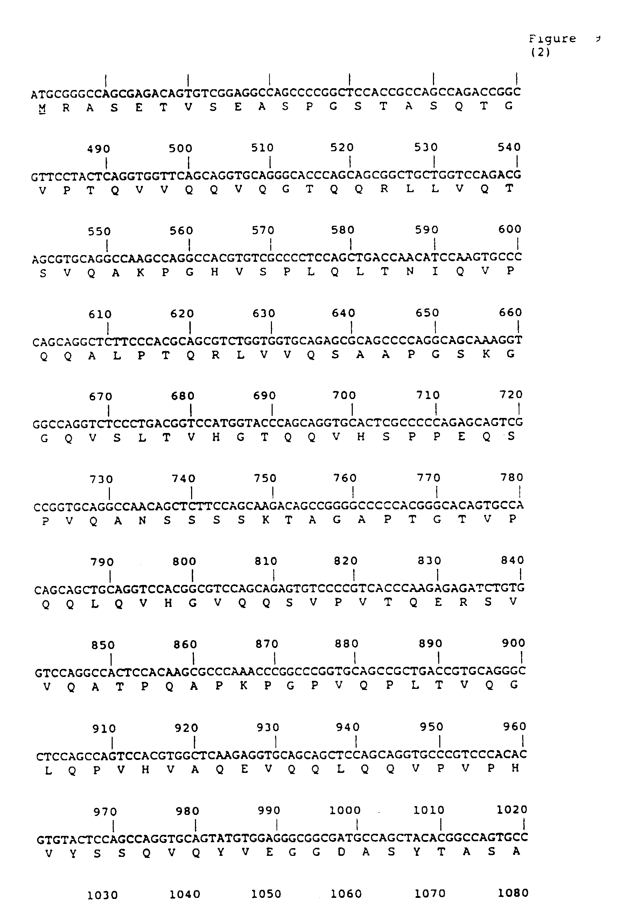

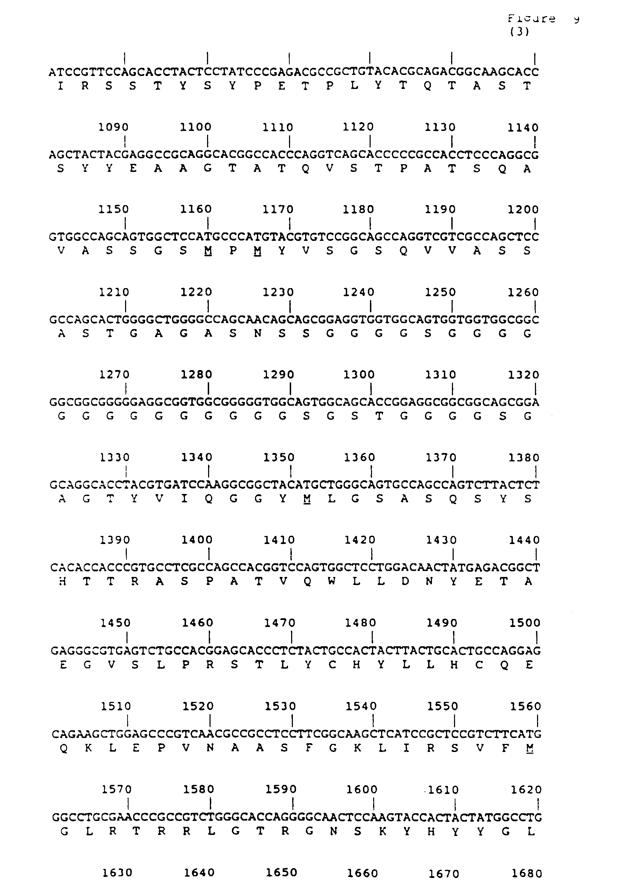

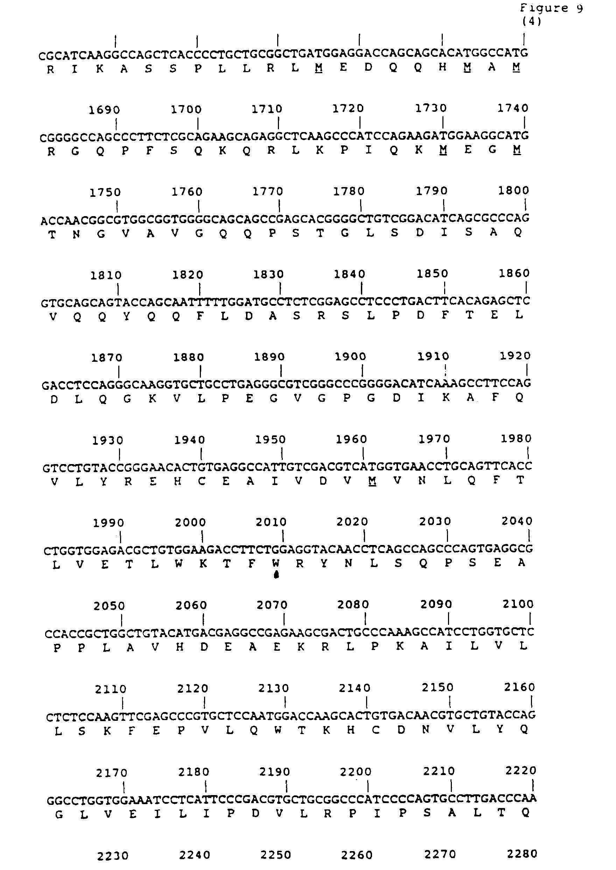

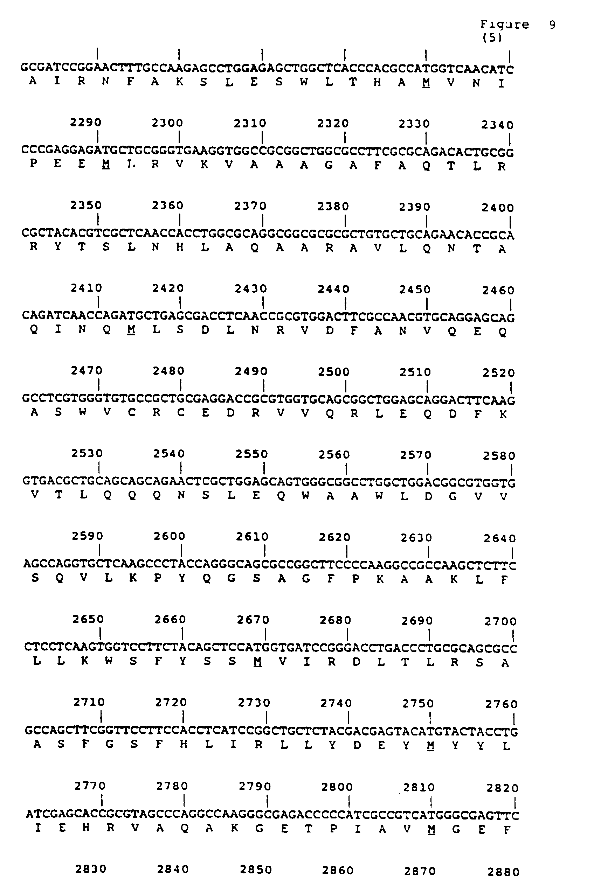

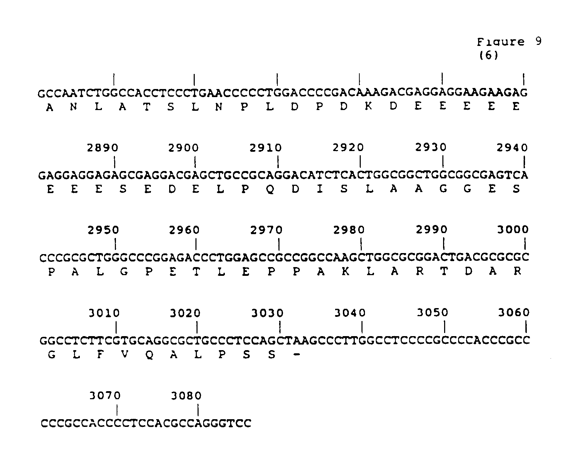

A particularly preferred embodiment of the present invention is the human RF-X protein. This protein naturally occurs for instance in human B lymphocytes and human fibroblastic cells. It is encoded by the DNA sequence given in Figure 9 (positions 94 to 3030).

Furthermore, truncated forms of the human RF-X protein are preferred embodiments of the present invention. The term "truncated" means that the protein lacks a certain segment of its amino acid sequence. DNA binding proteins involved in gene activation have two distinct portions or "domains". A DNA binding domain, responsible for interaction with DNA, and an activity domain, responsible for activation of the relevant gene. A "truncated" DNA binding protein, in which the activity domain has been partially or totally deleted, will still bind the specific DNA target sequence, however this binding will not result in gene activation. Moreover, such a "truncated" and inactive protein will occupy and block the binding site, will therefore prevent the binding of the normal active RF-X protein and consequently will function as an inhibitor of MHC class II gene expression. Pharmaceuticals capable of inhibiting or reducing MHC class II gene expression would be valuable in the treatment of autoimmune diseases or other conditions where the level of class II molecules should be lowered. The availability of the RF-X gene does provide the means to produce "truncated" forms of the RF-X protein with the ability to prevent the binding of the normal RF-X protein to DNA and thus with an MHC class II gene inhibitory activity.

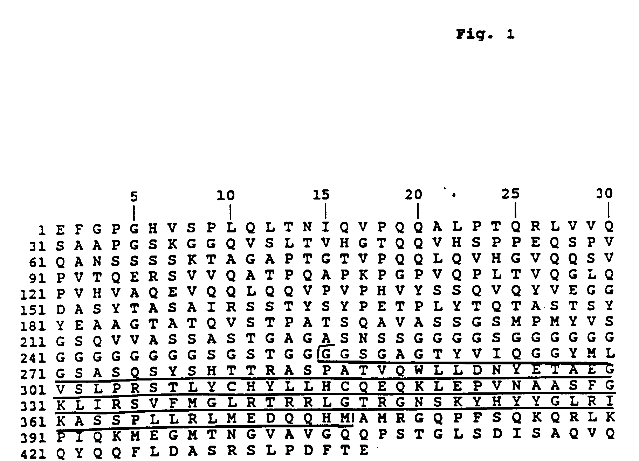

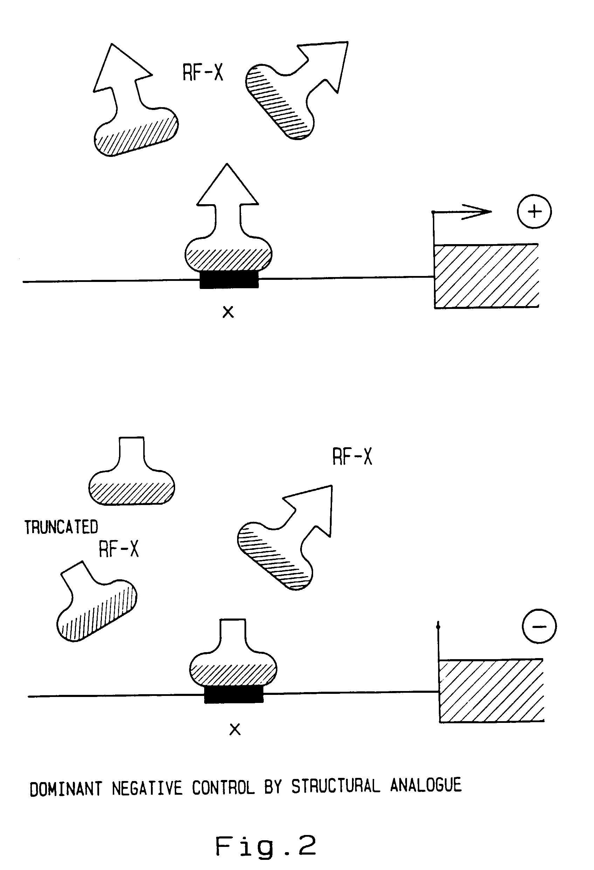

A particular preferred truncated form of the human RF-X protein has the amino acid sequence given in Figure 1. In comparison to the complete human RF-X protein, this truncated form lacks the entire COOH part of the protein but still contains the region responsible for binding to DNA. Another preferred truncated form of the human RF-X protein is a small sequence of 122 amino acids which is responsible for DNA binding (underlined in figure 1). This polypeptide, linked to any known nuclear targeting signal (such as that of SV 40 virus large T antigen), will constitute an even smaller "truncated" form of RF-X capable of inhibiting MHC class II gene expression. A nuclear targeting signal is a small segment of about 8 amino acids capable of directing a protein to the nucleus of cells. Such truncated forms of the RF-X protein can be used as pharmaceuticals. Figure 2 outlines the rational for the use of truncated forms of RF-X, or other DNA binding regulatory proteins, to compete for the binding of the normal protein and thus to inhibit MHC class II gene expression.

A further preferred embodiment of the present invention are proteins which substantially have the DNA binding characteristics of the naturally occurring protein but which are unable to activate the expression of MHC class II genes. Such proteins will also prevent the binding of RF-X to the promoters of MHC class II genes and behave as inhibitors of MHC class II gene expression. They could also be used as pharmaceuticals for that purpose.

A further preferred embodiment of the present invention is any protein capable of preventing the binding of the normal active RF-X protein to MHC class II promoter sequences, whether derived from the RF-X protein or not, and any other non-protein molecule with similar properties. These molecules, because of their capacity to inhibit RF-X binding to MHC class II promotors, will function in transcriptional intervention as inhibitors of MHC class II gene expression and be of value as pharmaceuticals. The availability of a recombinant RF-X protein will provide the means to assay for, search and identify molecules, including small molecules, possessing the same ability to prevent the binding of RF-X to the promoters of MHC class II genes and which could be used as pharmaceuticals as inhibitors of MHC class II gene expression in transcriptional intervention.

Furthermore, the present invention relates to the above mentioned proteins as product of recombinant DNA techniques.

Still further, the present invention relates to DNA sequences encoding the proteins of the present invention. Such DNA sequences can for instance be isolated from a cDNA library of recombinant bacteriophages, such as lambda gtll, constructed in such a way that the cDNA inserts are expressed in the form of their respective proteins (= "expression" library). The strategy for the identification of the bacteriophage clones expressing a given DNA binding protein consist in the screening of such an "expression" library with the relevant DNA target, radioactively labeled and used as a probe. In the case of factor RF-X, a DNA sequence containing the HLA-DRA X box, and known to bind natural factor X, was used as a probe to identify a recombinant bacteriophage clone expressing the DNA binding domain of RF-X. The insert of this recombinant clone is the DNA sequence which encodes most of protein RF-X.

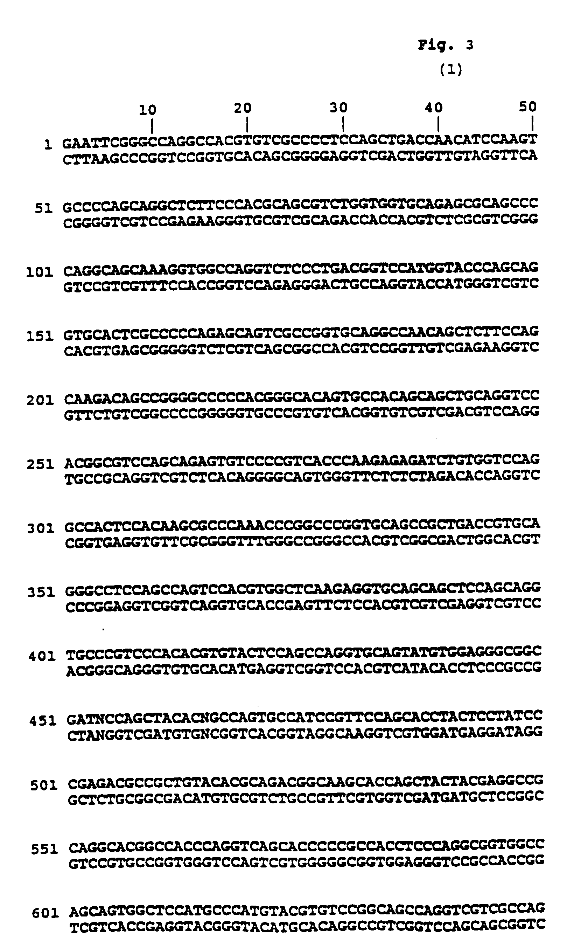

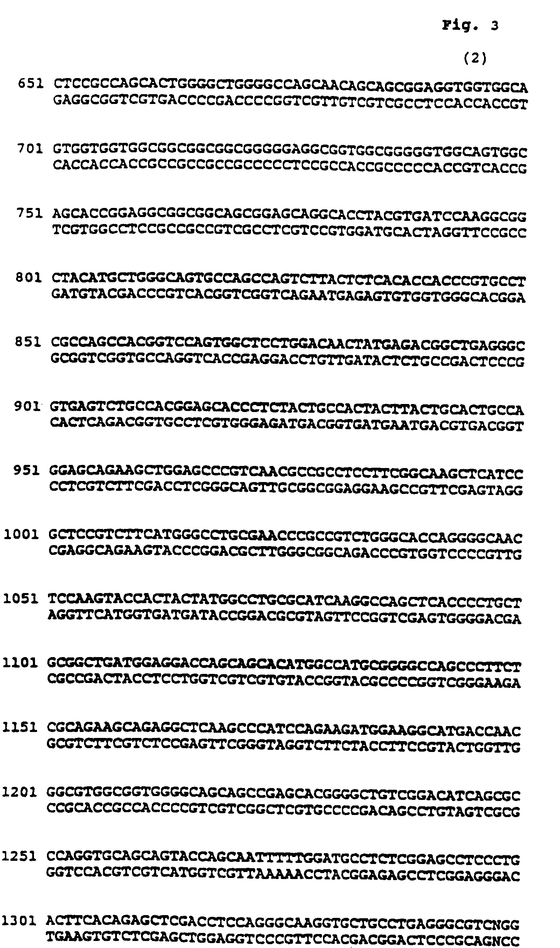

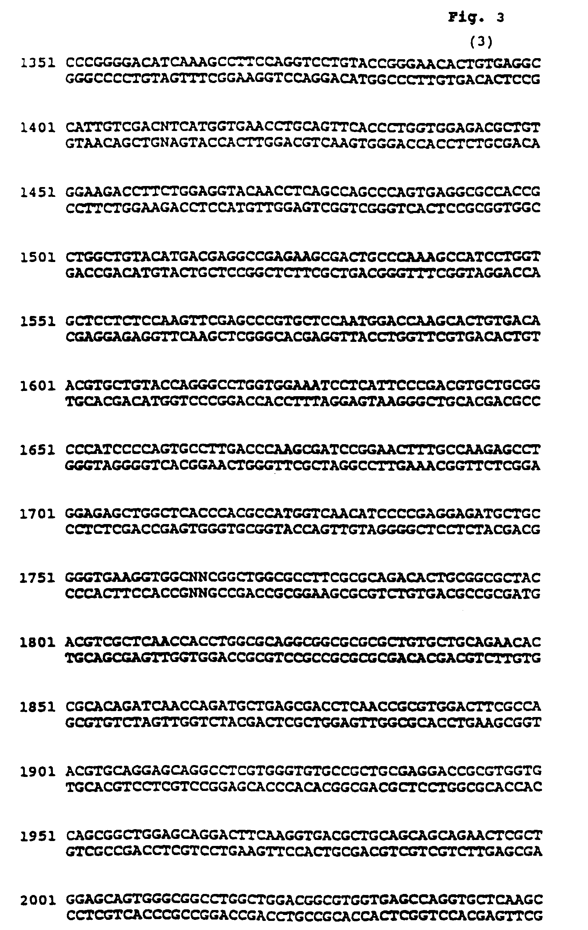

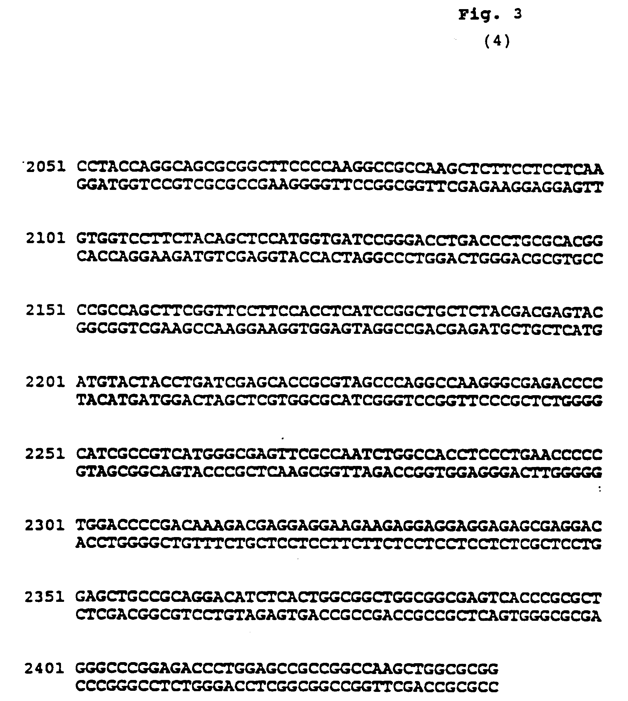

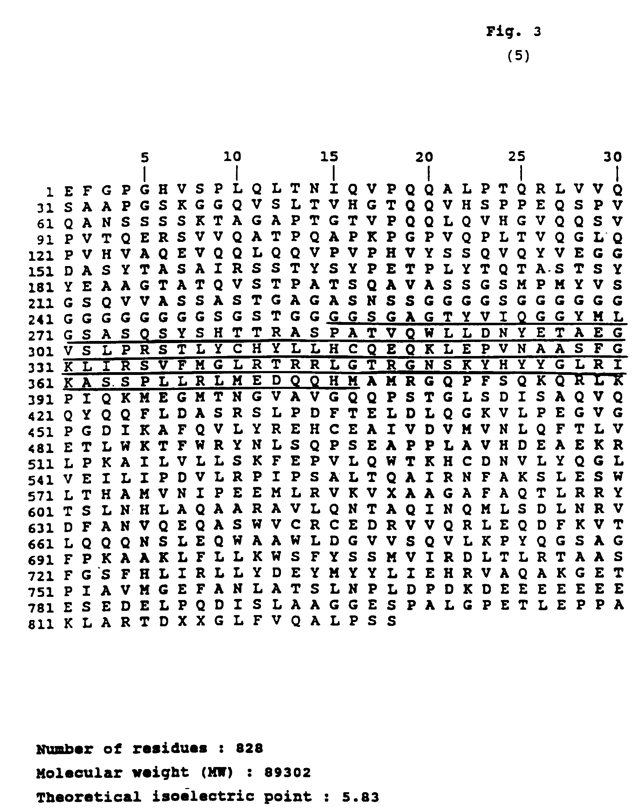

Preferred DNA sequence of the present invention are those given in Figure 3 or 9, which encode the above mentioned part of the human RF-X protein or the complete human RF-X protein, respectively. The coding region of the DNA seqeunce of Figure 9 is a particularly preferred DNA sequence of the present invention. It will be understood that the person skilled in the art provided with the DNA sequence of Figure 3 or 9 is in a position to use these DNA sequences, a fragment thereof or a corresponding oligonucleotide in order to isolate related DNA sequences from genomic or cDNA libraries of vertebrates, such as mammals or from other human individuals. Related DNA sequences are for instance allelic human DNA sequences.

On the other hand, the person skilled in the art provided with the screening strategy of the present invention is in a position to isolate corresponding DNA sequences from other vertebrates, such as mammals, or from other human individuals.

Furthermore, the DNA sequences given in Figure 3 or 9 or DNA sequences which are related to them can be obtained by chemical synthesis.

All the above DNA sequences are within the scope of the present invention. Thus, the present invention also relates to DNA sequences which hybridize to the DNA sequence of Figure 3 and/or Figure 9, preferably under conventional hybridization conditions. Conventional hybridization conditions are those where the Tm value is between about Tm-20 and Tm-27.

The present invention furthermore relates to DNA sequences which are related to the above DNA sequences by the degeneration of the genetic code.

In a further embodiment, the present invention relates to nucleotide sequences, i.e. to RNA or DNA sequences, which are complementary to the coding strand of the above mentioned DNA sequences. These complementary nucleotide sequences, also referred to as "antisense" RNA or DNA, are known to be capable of inhibiting the synthesis of the protein encoded by the relevant gene. The person skilled in the art and provided with the DNA sequences mentioned above will be in a position to produce and utilize the corresponding "antisense" RNA and DNA and to use them for the inhibition of synthesis of the RF-X protein and of related proteins. Inhibition of the synthesis of the RF-X protein, and of related proteins with a similar activity in the control of MHC class II gene expression, will result in the inhibition of the expression of MHC class II genes. Indeed, it is known from the study of mutant cells that a deficiency in RF-X is accompanied by a lack of expression of MHC class II genes (2). The use of "antisense" RNA and DNA molecules, as described above, as inhibitors of MHC class II gene expression will be important in medical conditions where a reduction of MHC class II molecules is desirable, as in the case of autoimmune diseases.

The invention also relates to fragments of said nucleotide sequences, preferably to fragments of their coding regions, including fragments of complementary or "antisense" RNA and DNA. The person skilled in the art and provided with the sequences described above is in a position to produce the corresponding short "antisense" oligonucleotides and to use them to achieve inhibition of RF-X synthesis and therefore inhibition of MHC class II gene expression. The person skilled in the art is also able to prepare chemically modified "antisense" molecules from the above sequences with longer half-lives and better ability to penetrate animal cells, as described in the literature.

Furthermore, the present invention relates to recombinant DNA molecules containing the DNA and nucleotide sequences of the present invention.

A preferred recombinant DNA molecule is a lambda gt11 bacteriophage containing, inserted in its unique EcoRI site, an almost full length cDNA encoding the human RF-X protein (see Figure 3), and referred to as λ9. Said cDNA was synthesized from polyA + RNA from human B lymphocytes known to express the RF-X factor. Another preferred recombinant DNA molecule is a pUC plasmid in which the RF-X cDNA described above was inserted at the unique EcoRI site of the plasmid, and referred to as pUC/λ9. This plasmid was deposited on April 13, 1989 under the requirements of the Budapest Treaty with the Deutsche Sammlung On Mikroorganismen, Braunschweig, Federal Republic of Germany, under the deposition number DSM 5303.

Another preferred recombinant DNA molecule is a Bluescript KS plasmid in which the full length RF-X cDNA (see Figure 9) was inserted between the unique EcoRI and HindIII sites, and referred to as pRF-XC. This full length RF-X cDNA was obtained by screening a cDNA library similar to that mentioned above and made from the same mRNA preparation. However, when preparing the first strand cDNA an oligonucleotide primer was used which is complementary to a sequence situated near the 5' end of pUC/λ9 (DSM 5303; nucleotides 301 to 341). Using standard hybridization techniques the cDNA clone RF-X5' that overlaps with the sequence of pUC/λ9 and corresponds to the remaining 5' end of the complete RF-X mRNA was isolated. The complete nucleotide sequence (and deduced amino acid sequence) of RF-X was derived from the overlapping of clones pUC/λ9 and RF-X5'. The complete RF-X cDNA was also reconstituted by an EcoRI - KpnI fragment from clone RF-X5' to a KpnI - HindIII fragment from clone pUC/λ9, thereby generating full length RF-X clone pRF-XC. An E. coli HB 101 strain harboring that plasmid and called pRF-XC, was deposited under the requirements of the Budapest Treaty on April 17, 1990 with the Deutsche Sammlung von Mikroorganismen, Braunschweig, Fed. Rep. of Germany, under the deposition number DSM 5876.

The invention also relates to host organisms which are transformed with the recombinant DNA molecules of the present invention. Preferably such host organisms are bacteria, such as E.coli or Bacillus subtilis, yeast, such as species or strains of Saccharomyces (e.g. Saccharomyces cerevisiae), and other eukaryotic host cells, such as insect or mammalian cells.

Furthermore, the present invention relates to processes for the production of proteins of the present invention comprising the steps of cultivating a transformed host organism of the present invention under suitable conditions in a culture medium and recovering the resulting expression product from the medium. Optionally, the recovered expression product can be purified according to conventional methods, such as chromatographies using ion exchange resins or affinity chromatography materials, e.g. monoclonal or polyclonal antibodies attached to a matrix.

Still further the present invention relates to pharmaceutical compositions which contain at least one of the proteins of the present invention or at least one of the nucleotide sequences of the present invention in a suitable dosage. These pharmaceutical compositions optionally also contain pharmaceutically acceptable carrier and/or additives.

The dosage depends on the condition of the patient and the symptoms of the disease.

In particular the pharmaceutical compositions of the invention containing the proteins of the present invention (except those which substantially have the DNA binding characteristics of the naturally occurring protein but which are unable to activate the expression of MHC class II genes) can be used for the treatment of diseases where an increase of the expression of MHC class II genes is desirable. Immunodeficiency due to a defect in the MHC class II regulatory factor RF-X corresponds to such a clinical condition. The mechanism of the beneficial effect will be by replacement of a defective RF-X protein in the patients.

The pharmaceutical compositions of the present invention which contain the proteins which substantially have the DNA binding characteristics of the naturally occurring protein, but which are unable to activate the expression of MHC class II genes, can be used for the treatment of diseases where a decrease of the level of the expression of MHC class II genes is desirable.

Among others, these include autoimmune diseases where an aberrant and excessive expression of HLA class II molecules at the surface of certain cells is thought to be responsible for the autoimmune pathological processes. These include Insulin Dependant Diabetes (IDD), Multiple Sclerosis (MS), Lupus Erythematosis (LE) and Rheumatoid Arthritis (RA). Aberrant expression of MHC class II genes and proteins has been documented in certain animal models of these diseases. Furthermore, animal models of some of these autoimmune diseases have been treated successfully with antibodies directed against MHC class II molecules, pointing to the desirability of downregulation the expression of MHC class II genes in autoimmune diseases.

The mechanism of inhibition of MHC class II genes by "truncated" forms of RF-X or related proteins, is based on the DNA binding domain of the "truncated" proteins competing with the naturally occurring active RF-X protein. It is discussed in greater details above (see also Figure 2).

The pharmaceutical compositions of the present invention which contain the above mentioned nucleotide sequence or a fragment thereof can also be used for the treatment of the above mentioned diseases where a decrease of the level of the expression of MHC class II genes is desirable. As discussed in details above, complementary or "antisense" RNA or DNA sequences will inhibit the synthesis of the protein RF-X, or of related proteins, and consequently inhibit the expression of MHC class II genes. A preferred way of achieving this inhibition in patients is through "antisense" oligonucleotides and one possible way of delivering these "antisense" oligonucleotides is in liposomes.

The present invention additionally relates to the use of the above proteins for the screening and identification of substances capable of inhibiting the expression of MHC class II genes. The above proteins, obtained as recombinant proteins, can be used by a person skilled in the art for the identification of substances capable of preventing the binding of the protein RF-X to the X box of the promoter of MHC class II genes, or the binding of other related proteins to other targets on the same promoters. This can be achieved by substances that interfer with or mimic the DNA binding domain of the protein or the target DNA sequence. This assay can be performed on a large scale screening as a search for substances with the above mentioned properties. The substances identified as capable of preventing the binding of RF-X in such an assay will be capable of inhibiting the expression of MHC class II genes. Their effects can then be tested in cell cultures and in vivo in animals.

The figures show:
**Figure 1:** A truncated form of protein RF-X still capable of binding the HLA class II promoter.
   Amino acid sequence of a form of protein RF-X where the COOH portion has been deleted. The underlined region corresponds to the DNA binding domain and a polypeptide consisting of that underlined sequence alone also binds to the X box of the HLA class II promoter.
**Figure 2:** Schematic representation of the rational for the inhibition of MHC class II gene expression by truncated forms of protein RF-X. Intact RF-X binds and activates MHC class II genes (top). Truncated form of RF-X competes for DNA binding with normal RF-X (bottom) and therefore inhibits gene expression.
**Figure 3:** Complete DNA sequence of the coding region of the cDNA insert of recombinant clone 9 encoding RF-X, with its corresponding amino acid sequence. The smallest amino acid sequence capable of binding to the HLA promoter X box is underlined.
**Figure 4:** A. Map of the HLA class II promoter with the target sequences for DNA binding factors, including the X box. B. Structure of the X1 and X3 oligonucleotides used for DNA binding and for the screening of RF-X encoding recombinant clones (nucleotide sequence and coordinate positions are from ref. 10).
**Figure 5:** Electrophoretic analysis of the proteins produced by the E. coli strain containing the recombinant expression plasmid pT7 /λ9. The major band around 90 kd corresponds to recombinant RF-X.
**Figure 6:** Nucleotide sequence of the coding part of vector pT7 /λ9 and corresponding amino acid sequence. An arrow indicates the first amino acid of the RF-X amino acid sequence.
**Figure 7:** Map of the different 3' and 5' deletions of the RF-X insert used to determine the location of the DNA binding domain of RF-X. The 122 amino acid segment of RF-X still capable of DNA binding is boxed.

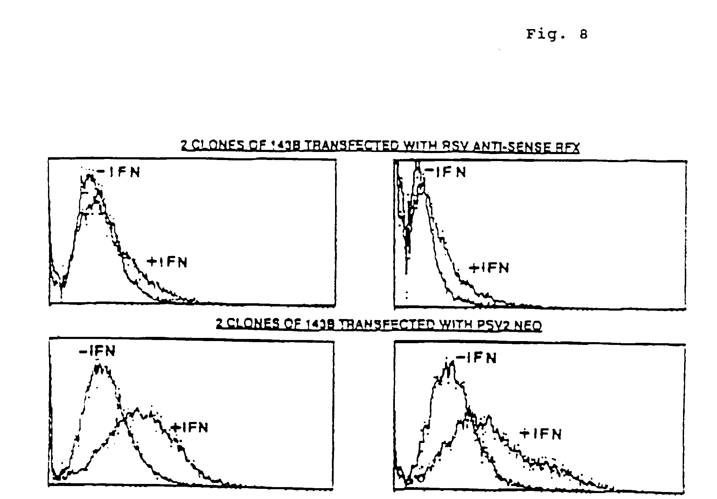

**Figure 8:** Down regulation of IFN-gamma induced HLA-DR expression by anti-sense RF-X.
   Fluorescence activated cell sorter (FACS) analysis of 143 B stable transfectant clones obtained either with RF-X antisense plasmids (top) or without (bottom). Following transfection and selection for G 418 resistance, cells were incubated with interferon gamma for 48 hrs. Then they were stained with L243, a specific monoclonal anti-DR antibody, and analyzed for HLA-DR expression at the cell surface by microfluorimetry. Control cells (bottom) express a normal amount of HLA-DR molecules, while RF-X antisense transfectants are inhibited in HLA-DR expression.
**Figure 9:** DNA sequence encoding the entire amino acid sequence of the human RF-X protein. The coding region begins at position 94 and extends to position 3030. Thus, the RF-X protein contains 979 amino acid positions. The DNA binding domain is represented by about amino acid position 406 to 527 whereas the activity domain is represented by about amino acid position 1 to 405. The amino acids are given in single letter code.

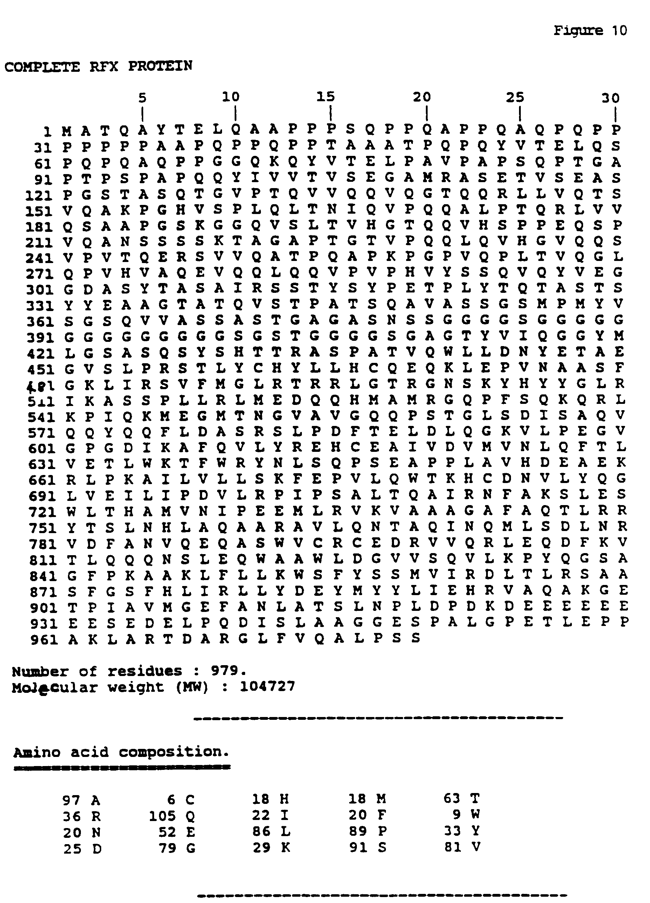

**Figure 10:** Amino sequence of the entire RF-X protein as encoded by the DNA sequence of Figure 9. Furthermore, the molecular weight and the amino acid composition are indicated.

The examples illustrate the invention. A general outline of the methods of molecular biology which can be applied in order to repeat the experiments described in the examples is given in Maniatis et al., "Molecular Cloning, A Laboratory Manual", Cold Spring Harbor Laboratory (1982) and (1989) 2nd Edition.

### EXAMPLE 1:

### Factor RF-X can bind to oligonucleotides X1 and X3.

The promoter of the DRA gene contains several conserved sequence motifs; a TATA box at position -24 to -28, an octamer motif at position -45 to -52, and two sequence elements called the class II X and Y boxes at positions -95 to -108 and -63 to -74 respectively (see Figure 4). Five distinct nuclear factors can be shown to bind to the DRA promoter. These include a factor binding to the X box (RF-X), a factor binding to the Y box (RF-Y), a factor binding to the octamer sequence (OBP), and two factors binding weakly to the spacer region between the X and Y boxes (NF-S) and to the W box, situated 5' of the X box (Figure 4).

RF-X is of particular interest for the regulation of class II gene transcription because it is absent or defective in inherited combined immunodeficiency (CID) (2), a disease characterized by a defect in a trans-acting regulatory factor controlling class II gene expression (1). Factor RF-X cannot bind to short oligonucleotides normally used for such DNA-binding studies but can be shown to bind to oligonucleotides containing sequences -70 to -144 (X1 oligo) or -70 to -124 (X3 oligo) of the DRA promoter. The structure of these oligonucleotide is given in Figure 4.

The following conditions were used to demonstrate this binding of RF-X : DNA oligonucleotide probes used for binding were labelled with (γ³²P)ATP and T4 polynucleotide kinase. Binding of 0.2-0.5 ng of end labelled DNA with 10 µg protein extract (prepared as described in ref 23) was done for 30 min at 0°C in 20 ll of 12% glycerol, 12 mM Hepes (pH 7.9), 60 mM KCl, 5 mM MgCl₂, 0.12 mM EDTA, 3.3 mM phenylmethylsulfonylfluoride, 0.3 mM dithiothreitol, 50 µg/ml poly(dIdC) /poly(dIdC) (Pharmacia), 25 µg/ml sonicated denatured E. coli DNA. Following binding, factor-DNA complexes were resolved from unbound DNA by non-denaturing polyacrylamide gel electrophoresis, performed as described (2).

The HLA-DQA and HLA-DPA X box oligonucleotides are identical to X3 with respect to length, and position of the X box, but cover sequences -119 to -173 and -143 to -197 of the HLA-DQA and HLA-DPA promoters respectively.

### EXAMPLE 2:

### Cloning of λ9, containing a cDNA encoding the human RF-X and subcloning of the cDNA insert.

A technique allowing the direct cloning of cDNA clones encoding sequence specific DNA-binding proteins has recently been developed; see (3),(4). The method involves screening a λgt11 cDNA expression library with a ³²P-labelled double stranded DNA probe containing the binding site for the protein of interest. The method is based on the fact that a cDNA encoding a given DNA binding protein inserted in λgt11 corresponds to a bacteriophage expressing that protein, or part of it, and that such a clone can be identified by the target DNA sequence used as a radioactive probe. Proteins from a large number of bacteriophage clones are transferred to membrane filters and the filters are probed with the radioactive specific DNA fragment, in this example a DNA fragment containing the DRA X box (namely oligonucleotide X1 of Figure 1). The sensitivity of the original method (3) has been considerably improved by two modifications, catenation of the probe to increase binding stability, and processing the filters through a guanidine hydrochloride denaturation/renaturation step prior to screening (4). Treatment of a B cell nuclear extract with guanidine hydrochloride has shown that RF-X is resistant to this denaturation/renaturation cycle. We therefore chose to adopt these modifications.

Total RNA was extracted from frozen cell pellets, prepared from a human B lymphocyte cell line (see Mann, (2)) grown in culture, using guanidium isothiocyanate essentially as described (5), and poly(A)⁺ RNA was purified by oligo(dT)-cellulose chromatography. Double stranded cDNA was synthesized by standard procedures (6) and cloned in kgt11 (7) using EcoRI linkers. A cDNA library containing 4x10⁶ clones was obtained. Plating, induction, transfer to nitrocellulose filters, and screening with ³²P-labelled probes were done exactly as described in (4). To prepare the X1 probe used during the primary screening, the X1 oligonucleotide (see Figure 4) was catenated by ligation to an average number of 10 and labelled by nick-translation. To prepare the non-specific pBR322 probe, a 75 bp HinfI fragment of pBR322 was treated with Klenow polymerase to generate blunt ends, and then catenated and labelled as described for the X1 probe. X3 and Xr probes (see Figure 4) were prepared by labelling the respective oligonucleotides with (γ³²P)ATP and T4 polynucleotide kinase, and subsequent catenation by ligation to an average number of 10.

750'000 recombinants were screened with a probe consisting of catenated and nick-translated X1 oligonucleotides. Of 20 phages selected for a second round of screening, only four were positive with the X1 probe and negative with a non-specific probe consisting of a catenated and nick-translated fragment of pBR322. This type of screening procedure is prone to artifacts such as the binding of proteins to single-stranded DNA or to the extremities of the probe. In addition, we know that at least one protein distinct from RF-X (NF-S) binds weakly to a sequence immediately 3' of the DRA X box. To eliminate such artifacts, a third round of screening was performed with two probes, a specific 54 bp long oligonucleotide centered on the X box (X3 in Figure 4, position -70 to -124 of the DRA promoter) and a non specific oligonucleotide which is identical to X3 except that a random 16 bp sequence replaces the X box. The Xr oligonucleotide is identical to X3 except that the X box (CCCCTAGCAACAGATG) is replaced with a random sequence (ATGTGTCTCGAACGCA). One of the four recombinant phages, λ9, was strongly positive with X3 and totally negative with Xr. Clone λ9 therefore encodes a protein that binds specifically to the X box. Interestingly, the guanidine hydrochloride denaturation/renaturation step used during the screening procedure proved to be essential for the identification of λ9, because when it was omitted the clone was no longer detectable with the specific X3 probe.

The 3.7 kb insert of clone λ9 was excised by digestion with EcoRI and subcloned into the plasmid vector pUC 18. This recombinant plasmid was called pUC/λ9. The recombinant plasmid pUC/λ9 was deposited with the Deutsche Sammlung von Mikroorganismen (DSM) in Braunschweig, Fed. Rep. of Germany under the deposition number DSM 5303.

### EXAMPLE 3:

### Characterization of the recombinant protein encoded by λ9

Isolation and induction of λgt11 and λ9 lysogens (4), and preparation of bacterial extracts (3 ) were done as described. Lysogens of λgt11 and λ9 were isolated and induced to express their respective β-galactosidase genes. Protein extracts were prepared from the lysogens and from uninfected host bacteria. The extracts were then analyzed for X box binding activity by means of a gel retardation assay (2). With the λ9 extract, one major protein-DNA complex is formed with oligonucleotide X1. It is specific to the λ9 extract since it is not observed with extracts from either a λgt11 lysogen or the host bacterial strain. The 9-encoded protein also binds to the smaller X3 oligonucleotide but not to the control Xr probe, confirming that λ9 encodes a true X box binding protein.

The binding site and the contact points of the recombinant protein were further analyzed by methylation interference analysis. The X1 oligonucleotide was subcloned into the Smal site of pUC12, cut out with EcoRI and HindIII, and labelled at the EcoRI site using (α³²P)ATP and T4 polynucleotide kinase for the coding strand or (α³²P)dATP and Klenow polymerase for the non-coding strand. Methylation of probe, binding with a protein extract from clone λ9, purification of free and bound DNA, and sequence gel electrophoresis were done as described in (2). The X1 oligo was end-labelled on the coding or non-coding strand, partially methylated with dimethylsulfate, and incubated with the λ9 lysogen extract. After gel purification of free and complexed fragments, DNA was cleaved at methylated G residues and analyzed by sequencing gel electrophoresis. Comparison of the cleavage profiles obtained with free and bound DNA reveals a methylation interference pattern strikingly similar to the one observed with native RF-X (2). All five contact points, including the G residue at which methylation enhances binding, are shared by both RF-X and the recombinant protein.

### EXAMPLE 4 :

### Clone λ9 corresponds to a single copy gene

Genomic DNA from human B cell lines was analyzed by Southern blotting, performed according to standard procedures. DNAs extracted from two normal B cell lines were digested with three restriction enzymes, fractionated by gel electrophoresis, transferred to filters, and hybridized with ³²P-labelled probes consisting either of the entire 3.7 kb insert of clone λ9, or of a 600 bp SmaI-EcoRI fragment derived from its 3' end.

The hybridization pattern obtained with the two DNAs are identical and show few prominent bands when the entire cDNA insert is used as probe. For each enzyme, the 600 bp subfragment hybridizes to only one of the genomic fragments. Finally, only two genomic Hind III fragments are detected and the RF-X cDNA contains a Hind III site. These results indicate that the cDNA we have isolated corresponds to a single-copy gene.

The RF-X gene is highly conserved in mouse DNA and the restriction pattern of the mouse RF-X homologue is clearly different from that of other genes recently described (8) and thought to encode DNA binding proteins. RF-X is therefore distinct from these other proteins.

### EXAMPLE 5 :

### RF-X mRNA is rare and is expressed normally in the regulatory mutants

We investigated the presence of RF-X mRNA in these mutants by Northern blot and RNAse protection experiments. Analysis of poly(A)⁺RNA by Northern blot hybridization (3) was done as described. Probes used for filter hybridizations were labelled with (α³²P)dCTP by random primed synthesis. Poly(A)⁺ RNA from a normal and two CID B cell lines was fractionated by gel electrophoresis, transferred to a nitrocellulose filter and hybridized with the 3.7 kb insert of λ9. A 4.1 kb mRNA is detected in both the normal and mutant cells.

The presence of RF-X mRNA was further analyzed by means of an RNAse protection experiment. The cDNA insert of λ9 was subcloned in a Bluescript vector (Stratagene). The plasmid was digested with Fnu4HI and transcribed with T3 RNA polymerase in the presence of (α³²P)UTP. This generates a 139 nucleotide probe that is homologous to 71 nucleotides located at the extreme 3' end of the mRNA corresponding to λ9. Hybridization of the probe with poly(A)⁺RNA, digestion with RNAse A and T1, and denaturing gel electrophoresis were done as described in (9). The exposure time required indicates that RF-X mRNA is present at a very low steady state level. The probe is protected to the same extent by RNA from normal and CID B cells. Thus, RF-X mRNA is normal in both size and abundance in the mutant cells.

### EXAMPLE 6 :

### RF-X has different binding affinities for the promoter squences of HLA-DRA, HLA-DQA and HLA-DPA.

Native RF-X can bind to the X boxes of all class II genes that have been analyzed. These include the human DRA, DRB1, DRB3, DPA and DQA genes, the mouse Ea and Eb genes. The binding of RF-X to the X box motive of different HLA clas II genes was compared. Using oligonucleotides specific of HLA-DRA, -DQA and -DPA, similar in length to X3 in Figure 4 (54 base pairs) and all centered on the MHC class II X box, it can be shown that natural RF-X (from nuclear extracts) binds strongly to DRA, less to DPA and much less to DQA. RF-X has therefore strikingly different affinities for the X boxes of HLA-DRA, -DPA, and -DQA genes. Therefore, relative binding affinity of the recombinant protein for the X boxes of these three genes was analyzed by competition experiments. The λ9 extract and a B cell nuclear extract were incubated with the ³²P-labelled X1 oligonucleotide in the presence of different amounts of unlabelled DRA, DQA or DPA competitor oligonucleotides, and the extent of competition was analyzed by gel electrophoresis. RF-X and the protein encoded by λ9 have the same relative binding affinities for the three X boxes. In both cases, binding is competed out efficiently by DRA even at a low molar excess of competitor, only very poorly by DQA even at a high molar excess of competitor, and at an intermediate efficiency by DPA. Competition with Xr has no effect on binding of either RF-X or the recombinant protein. This provides an additional evidence for the fact that clone λ9 encodes RF-X. The DNA-binding assays used to demonstrate these different affinities are those described in Example 1.

### EXAMPLE 7 :

### Determination of the DNA sequence of the 3.7 kb insert of the cDNA clone λ9 and identification of the coding region.

The insert of clone λ9 was recovered by digestion with EcoRI and subcloned in the M13 bacteriophage. The complete DNA sequence of the coding region was determined by the method of dideoxy sequencing and of Maxam-Gilbert (see Figure 3). The longest open reading frame contained in that sequence encodes 828 amino acids, terminating with a stop codon. The 3.7 kb insert consists of 2540 nucleotides encoding 828 amino acids, followed by about 1260 nucleotides of the 3' untranslated region. The 3.7 kb insert does not contain the initiation codon AUG and the 5' untranslated region.

### EXAMPLE 8 :

### Expression of the λ9 insert and production of recombinant proteins in E.coli.

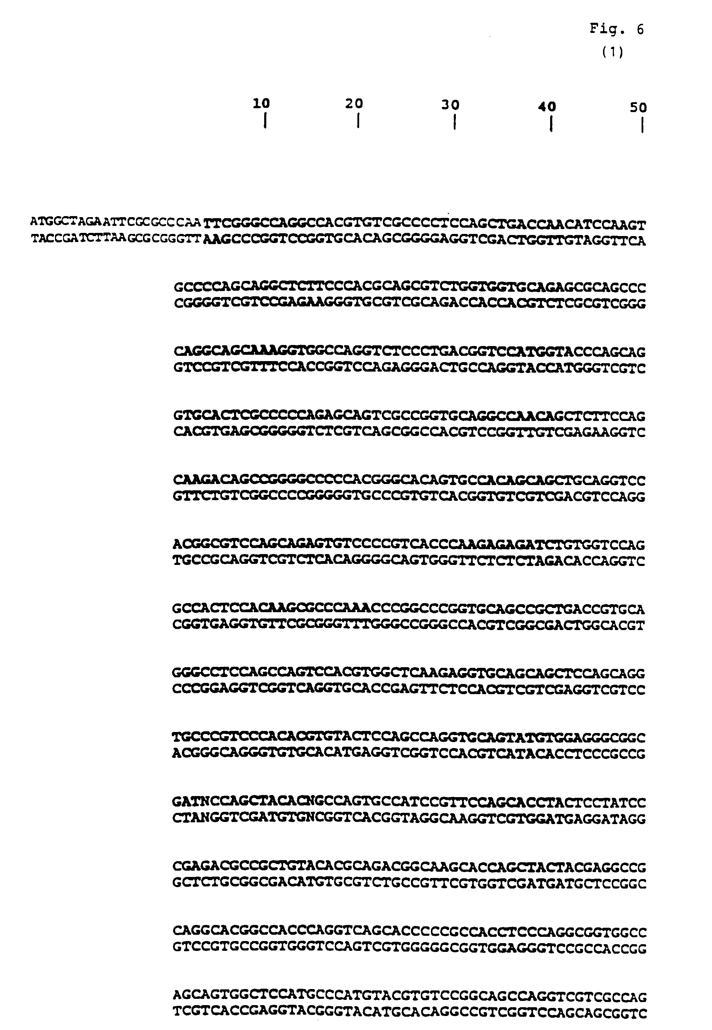

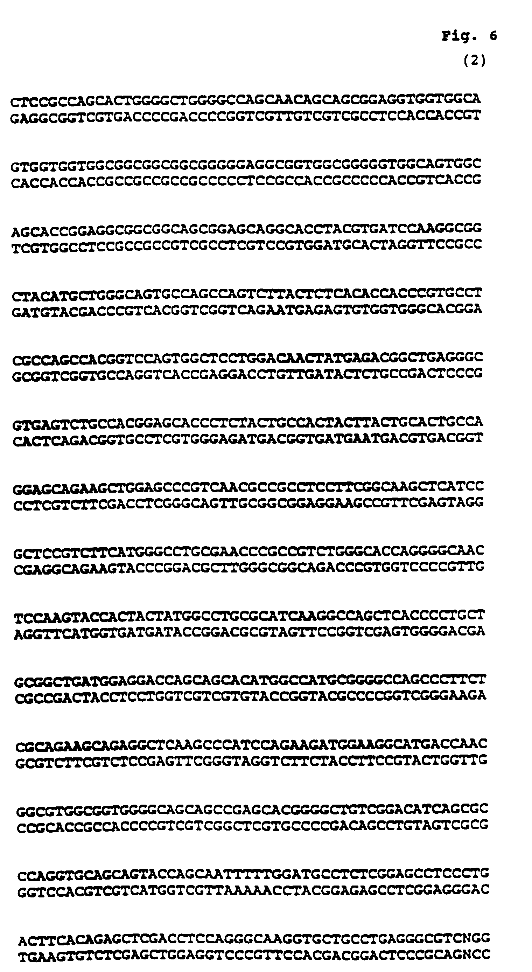

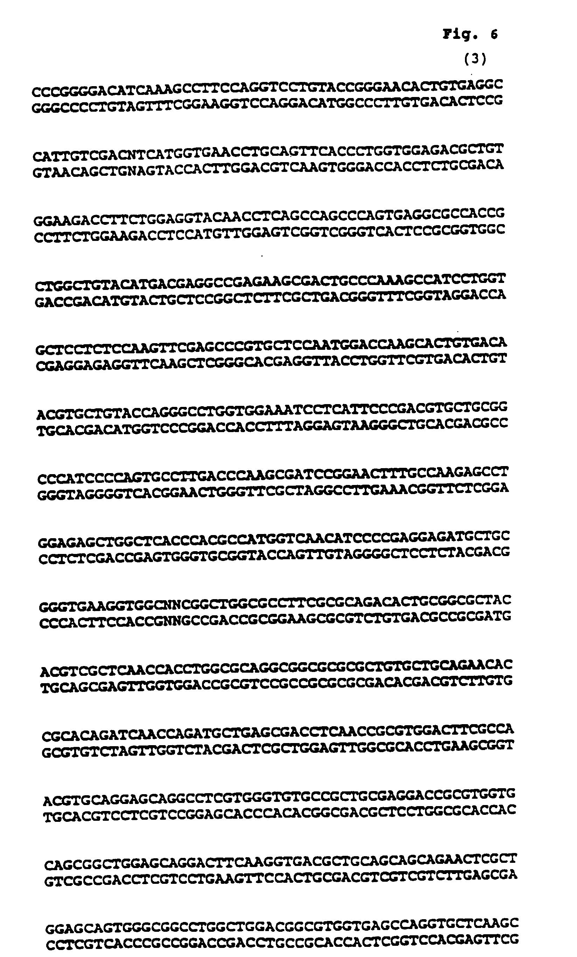

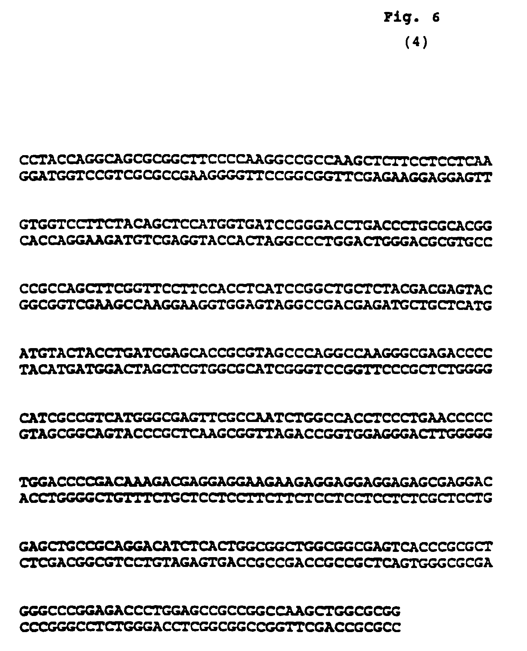

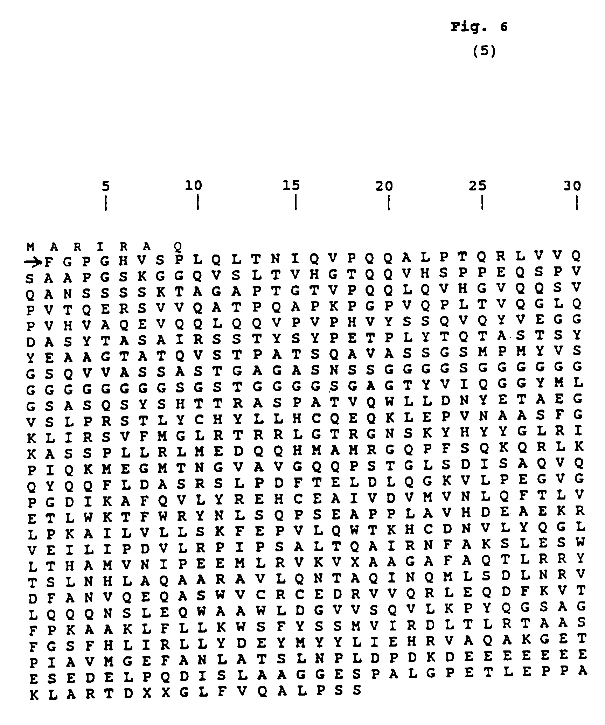

The 3.7 kb EcoRI insert was subcloned in phase in the E.coli expression vector pT7/7. This expression vector is derived from plasmid pBR322 and contains the T7 phage promoter described in (11) followed by an AUG codon and a short DNA sequence containing several restriction sites. Knowing the sequence and the reading frame of both the insert and the vector sequence allowed the subcloning in the correct reading frame. The resulting recombinant expression vector was called pT7/λ9. The DNA sequence of the complete coding region is given in Figure 6. Following culture of recombinant bacteria harboring the recombinant expression plasmid pT7/λ9, bacterial pellets were extracted with 6 M guanidine hydrochloride, fractionated on a sizing column (Sephacryl) in guanidine hydrochloride and aliquots were analyzed on PAGE gels.

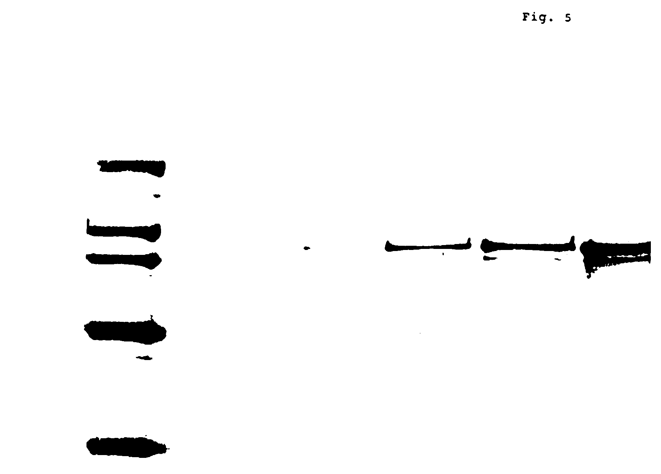

Figure 5 shows the protein staining of these gels. The proteins expressed correspond to the size expected from the 828 amino acid open reading frame of the λ9 insert.

As already mentioned above, Figure 6 shows the DNA sequence of the coding part of vector pT7/λ9 and the amino acid sequence of the expressed recombinant RF-X protein. The first 6 amino acids are from the pT7/7 vector, amino acid 7 is from the "in phase" ligation used (Smal and blunt ended EcoRI) and amino acid 8 is the beginning of the RF-X sequence.

### EXAMPLE 9 :

### Expression of truncated forms of the RF-X recombinant protein.

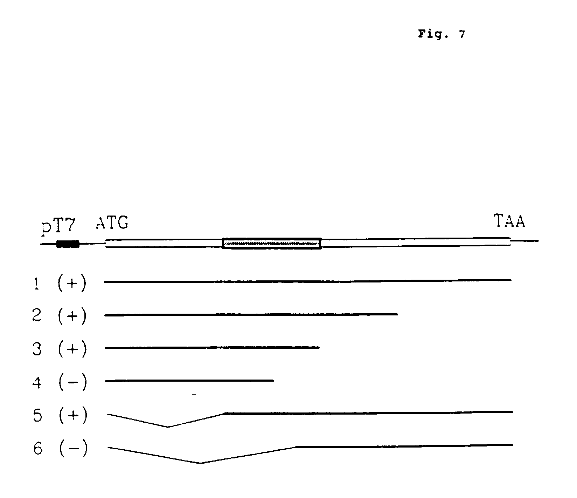

The purpose of this example is to identify the location of the DNA binding domain of RF-X and to test for the ability of smaller forms of the RF-X protein to bind its target sequence, the HLA-DRA X box. The λ9 insert of vector pT7/λ9 is transcribed in vitro into RNA with T7 polymerase and, following capping, the RNA transcript is translated in a reticulocyte lysate system, using standard reagents. The in vitro made protein is tested for its specific binding to the X3 oligo, under the conditions described in Example 1. In order to generate truncated forms of RF-X, the 3.7 kb insert of clone λ9 is first cleaved with various restriction enzymes so as to remove various portions of the 3' end of the insert. The fragments obtained are then subcloned in phase in the pT7-7 vector, as mentioned above. RNA is synthesized in vitro and the different proteins synthesized from in vitro made RNA correspond to progressive deletions of the COOH end of RF-X. When these truncated forms of RF-X are made and tested for binding to the X box of the HLA-DRA promoter, one observes that the NH2 half of RF-X is still binding to DNA. Further deletions of the 3' as well as the 5' portions of the RF-X coding region (Figure 7) reveal a 122 amino acid long segment (underlined in Figure 1 and Figure 6) which is responsible for DNA binding and which by itself can still bind to the correct HLA promoter X box DNA target oligonucleotide.

This demonstrates that a very short segment of protein RF-X can be produced and is still capable of binding to HLA class II promoters. Such smaller forms of RF-X, capable of DNA binding but inactive in Class II gene activation, should behave as dominant repressor molecules and inhibit HLA class II gene expression. It is expected on that basis that such molecules act as inhibitors of HLA class II gene expression.

### EXAMPLE 10 :

### Recombinant RF-X can be used to screen for inhibitors of RF-X binding.

It might be desirable to identify compounds that inhibit the binding of RF-X to its target DNA sequence. Such molecules would behave as inhibitors of HLA class II gene expression. One way demonstrated here consist in using the DNA binding assay (see Example 1) with recombinant RF-X as a substrate for this binding. As an example of the identification of such compounds by that procedure, when various oligonucleotide preparations are tested for their ability to inhibit RF-X binding, oligonucleotides such as X1 or X3 (see above) are effective inhibitors. The same assay making use of recombinant RF-X can therefore be applied by someone skilled in the art to the systematic testing and screening of other substances as inhibitors of RF-X binding.

### EXAMPLE 11 :

### Inhibition of the synthesis of RF-X by antisense RNA.

One way to modulate HLA class II gene expression is to inhibit the biosynthesis of the RF-X protein. In this Example, RF-X is synthesized in vitro in a rabbit reticulocyte lysate system with in vitro synthesized RF-X RNA as template, as described above in Example 9. In addition, the anti-sense form of RF-X RNA is prepared in vitro with T7 polymerase from a pT7 plasmid into which the RF-X insert was subcloned in the opposite orientation. When the RF-X anti-sense RNA is added to the cell free system together with RF-X RNA template, the synthesis of the RF-X protein is inhibited. This shows that RF-X antisense RNA can effectively inhibit the synthesis of the RF-X protein and suggests that antisense DNA will have a similar effect. When applied to intact cells, antisense RF-X RNA or DNA should therefore inhibit the synthesis of endogenous RF-X protein and consequently result in a down regulation of the expression of MHC class II genes.

### EXAMPLE 12 :

### Inhibition of HLA class II expression by RF-X antisense in intact cells.

Plasmids were constructed with various segments of RF-X cDNA inserted under the control of a promoter, but in the opposite orientation (3' to 5'). This was achieved by standard procedures of cleavage and ligation, making use of generally available vectors. A human fibroblastic cell line (143 B) was transfected with a plasmid encoding resistance to the drug G 418 ("Neomycin resistance" gene), with or without the RF-X antisense plasmid. G 418 resistant stable transfectants were selected and analysed for their ability to express HLA class II molecules in response to stimulation by interferon gamma.
The fluorescence profile of such cells, analysed with a monoclonal antibody specific of HLA-DR is presented in the Figure. Control transfectants (with neomycin resistance alone) respond well and express HLA-DR as expected. Transfectants containing RF-X antisense plasmids are strongly inhibited in their HLA class II response. This inhibition was confirmed at the level of HLA-DR mRNA by hybridisation experiments. We can conclude that we have demonstrated the use of RF-X antisense as an agent capable of inhibiting the expression of HLA class II genes and molecules in intact cells.

### EXAMPLE 13: Determination of the sequence of the 5' portion of the RF-X CDNA

Because the RF-X cDNA clone pUC/λ9 was incomplete at its 5' end, the above-mentioned preparation of B cell mRNA was again used to obtain the remaining 5' portion of RF-X cDNA. The 5' portion of RF-X cDNA was synthesized with reverse transcriptase from B cell mRNA utilizing as primer an oligonucleotide derived from the 5' region of the sequence of the incomplete pUC/λ9 RF-X cDNA. That oligonucleotide is complementary to positions 301 to 321 of the pUC/λ9 cDNA sequence shown in Fig. 3. The cDNA was synthesized, ligated to λgt11 phage arms, packaged and spread onto standard culture plates exactly as described in Example 2. Transfer to nitrocellulose membranes, prehybridization and hybridization were performed by standard procedures (Maniatis et al., supra). As probe, an EcoRI - KpnI fragment corresponding to positions 1 to 141 in Figure 3 and labelled with ³²P by random priming was used. This yielded a clone (called RF-X5') that showed a DNA sequence overlapping on its 3' part with the 5' part of the incomplete RF-X pUC/λ9 cDNA, and corresponding therefore to the missing 5' portion of the complete RF-X cDNA. The sequence of the entire RF-X cDNA, as derived from both overlapping clones, is presented in Figure 9. The 3' end of RF-X cDNA, including the 3' untranslated region was now determined unequivocally down to position 3086.

In order to reconstruct a full length RF-X cDNA, a standard ligation was performed, taking advantage of a unique KpnI site present in both RF-X5' and pUC/λ9 cDNA clones, and located on position 141 of the sequence of clone pUC/λ9. An EcoRI - KpnI fragment from RF-X5' was ligated to a KpnI - HindIII fragment from clone pUC/λ9, and the resulting full-length RF-X cDNA was then subcloned into the standard plasmid vector Bluescript KS, generating plasmid pRF-XC. An E. coli strain harboring this RF-X full-length plasmid (pRF-XC) was deposited at the Deutsche Sammlung von Mikroorganismen, Braunschweig, Fed. Rep. of Germany, on April 17, 1990 under the deposition number DSM 5876.

### EXAMPLE 14: Use of recombinant RF-X protein in a screening assay to identify inhibitors of RF-X binding and of MHC class II expression.

With the use of recombinant RF-X, or truncated forms of recombinant RF-X containing its DNA binding domain, standard binding assays can be set up as a test for potential inhibitory molecules. The binding assay is either the "gel retardation assay" (or "band shift" assay) or the immunoprecipitation of the RF-X/DNA complex with an anti-RFX antibody. A large number of different molecules can thus be screened for their ability to inhibit the binding of RF-X and consequently for their ability to inhibit the expression of MHC class II genes.

### References :

1. de Préval, C., Lisowska-Grospierre, B., Loche, M., Griscelli, C. & Mach, B. (1985) Nature **318**, 291-293.
2. Reith, W., Satola, S., Herrero Sanchez, C., Amaldi, I., Lisowska-Grospierre, B., Griscelli, C., Hadam, M. R. & Mach, B. (1988) Cell **53,** 897-906.
3. Singh, H., LeBowitz, J. H., Baldwin, A. S. Jr & Sharp, P. A. (1988) Cell **52,** 415-423.
4. Vinson, C. R., LaMarco, K.L., Johnson, P. F., Landschulz, W. H. & McKnight, S. L. (1988) Genes and Development 1, 801-806.
5. Chirgwin, J. M., Przbyla, A. E., MacDonald, R. J. & Rutter, J. (1979) Biochem. **18,** 5294.
6. Gubler, U. & Hoffman, B. J. (1983) Gene **25**, 263-270.
7. Huynh, T. V., Young, R. A. & Davies, R. W. (1985) in DNA Cloning: a Practical Approach, ed. Glover, D. M. (IRL Press, Oxford, Washington) Vol. 1, 49-77.
8. Liou, H., Boothby, M. R. & Glimcher, L. H. (1988) Science **242**, 69-71.
9. Bellocq, C. & Kolakofsky,D. (1987) J. Virol. **61**, 3960-3967.
10. Schamboeck, A., Korman, A.J., Kamb, A. and Strominger, J.L. Nucl.Acid.Research, (1983) 11, 8663 .
11. Rosenberg, A.H., Lade, B.N., Chin, D., Lin, S-W., Dunn, J.J. and Studier, F.W., Gene 56(1987), 125-135.

## Claims (Claims for the following Contracting State(s): AT, BE, CH, DE, DK, FR, GB, IT, LI, LU, NL, SE)

1. Protein which naturally occurs in vertebrate cells expressing MHC class II genes, regulates the expression of vertebrate MHC class II genes, and binds to the X box of DNA sequences controlling the expression of said MHC class II genes.

2. Protein according to claim 1 which naturally occurs in mammalians, preferably in humans.

3. Protein according to claim 1 or 2 which has the biological activity of the RF-X protein which naturally occurs in human cells expressing MHC class II genes and which binds to oligonucleotides containing the sequences -70 to -144 (X1) or -70 to -124 (X3) of the DRA promoter shown in Figure 4.

4. protein according to any one of claim 1 to 3 which is the human RF-X protein.

5. Protein according to claim 3 which is a truncated form of the human RF-X protein.

6. Protein according to claim 3 or 5 which has the amino acid sequence : 
5 10 15 20 25 30
1 E F G P G H V S P L Q L T N I Q V P Q Q A L P T Q R L V V Q
31 S A A P G S K G G Q V S L T V H G T Q Q V H S P P E Q S P V
61 Q A N S S S S K T A G A P T G T V P Q Q L Q V H G V Q Q S V
91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S M P M Y V S
211 G S Q V V A S S A S T G A G A S N S S G G G G S G G G G G G
241 G G G G G G G S G S T G G G G S G A G T Y V I Q G G Y M L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V M G E F A N L A T S L N P L D P D K D E E E E E E E
781 E S E D E L P Q D I S L A A G G E S P A L G P E T L E P P A
611 K L A R T D X X G L F V Q A L P S S
 or the amino acid sequence: 
5    10    15    20    25    30
1 M A T Q A Y T E L Q A A P P P S Q P P Q A P P Q A Q P Q P P
31 P P P P P A A P Q P P Q P P T A A A T P Q P Q Y V T E L Q S
61 P Q P Q A Q P P G G Q K Q Y V T E L P A V P A P S Q P T G A
91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 wherein the amino acid sequence having 979 positions represents the entire human RF-X protein.

7. Protein according to any one of claim 1 to 6 which substantially has the DNA binding characteristics of the naturally occurring protein but which is unable to activate the expression of the MHC class II genes.

8. Protein according to any one of claims 1 to 7 which is a recombinant protein.

9. DNA sequence encoding a protein according to any one of claims 1 to 8.

10. DNA sequence according to claim 9 which encodes an incomplete form of the human RF-X protein comprising the following nucleotide positions :

                10        20        30        40        50
                 |         |         |         |         |
  1 GAATTCGGGCCAGGCCACGTGTCGCCCCTCCAGCTGACCAACATCCAAGT
    CTTAAGCCCGGTCCGGTGCACAGCGGGGAGGTCGACTGGTTGTAGGTTCA

 51 GCCCCAGCAGGCTCTTCCCACGCAGCGTCTGGTGGTGCAGAGCGCAGCCC
    CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
    GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
    CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
    GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
    TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
    CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
    CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
    ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
    CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
    GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
    GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
    TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGAGGCGGTGGCGGGGTGGCAGTGGC
CACCACCACCGCCGCCGCCGCCCCTCCGCCACCGCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
     GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
     GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
     CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
     GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
     CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
     TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
     GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
     CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
     CCCACTTCCACCGNNGCCGACCGCGGAAGCGCGTCTGTGACGCCGCGATG

1801 ACGTCGCTCAACCACCTGGCGCAGGCGGCGCGCGCTGTGCTGCAGAACAC
     TGCAGCGAGTTGGTGGACCGCGTCCGCCGCGCGCGACACGACGTCTTGTG

1851 CGCACAGATCAACCAGATGCTGAGCGACCTCAACCGCGTGGACTTCGCCA
     GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
     TGCACGTCCTCGTCCGGAGCACCCACACGGCGACGCTCCTGGCGCACCAC

1951 CAGCGGCTGGAGCAGGACTTCAAGGTGACGCTGCAGCAGCAGAACTCGCT
     GTCGCCGACCTCGTCCTGAAGTTCCACTGCGACGTCGTCGTCTTGAGCGA

2001 GGAGCAGTGGGCGGCCTGGCTGGACGGCGTGGTGAGCCAGGTGCTCAAGC
     CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGAGCGAGGAC
ACCTGGGGCTGTTTCTGCTCCTCCTTCTTCTCCTCCTCCTCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. DNA sequence according to claim 9 which encodes the entire human RF-X protein comprising the following nucleotide positions: 
10        20        30        40        50
|         |         |         |         |
1 GCGAAAAGCGTTTCCGCAGACAGAAGTGGGGAGAAGCGGAGGAATTAAAA
CGCTTTTCGCAAAGGCGTCTGTCTTCACCCCTCTTCGCCTCCTTAATTTT

51 AAAAAAAAAAGCCTTATTTATTATCATTTTCCCCACCGTTGGCATGGCAA
TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
GTCGTCCGAGAAGGGTGCGTCGCAGACCACCACGTCTCGCGTCGGGGTCC
 
651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG

701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGAGGCGGTGGCGGGGGTGGCAGTGGCAGCA
ACCACCGCCGCCGCCGCCCCTCCGCCACCGCCCCCACCGTCACCGTCGT

1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG

1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG
 
1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1801 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCCG
GTGTCTCGAGCTGGAGGTCCCGTTCCACGACGGACTCCCGCAGCCGGGGC

1901 GGGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT

2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC

2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA
 
2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCGCTGTGCTGCAGAACACCGCA
GCGAGTTGGTGGACCGCGTCCGCCGCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG

2701 GCCAGCTTCGGTTCCTTCCACCTCATCCGGCTGCTCTACGACGAGTACAT
CGGTCGAAGCCAAGGAAGGTGGAGTAGGCCGACGAGATGCTGCTCATGTA

2751 GTACTACCTGATCGAGCACCGCGTAGCCCAGGCCAAGGGCGAGACCCCCA
CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
AGCGGCAGTACCCGCTCAAGCGGTTAGACCGGTGGAGGGACTTGGGGGAC

2851 GACCCCGACAAAGACGAGGAGGAAGAAGAGGAGGAGGAGAGCGAGGACGA
CTGGGGCTGTTTCTGCTCCTCCTTCTTCTCCTCCTCCTCTCGCTCCTGCT
 
2901 GCTGCCGCAGGACATCTCACTGGCGGCTGGCGGCGAGTCACCCGCGCTGG
CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
CCGGAGAAGCACGTCCGCGACGGGAGGTCGATTCGGGAACCGGAGGGGCG

3051 CCCACCCGCCCCCGCCACCCCTCCACGCCAGGGTCC
GGGTGGGCGGGGGCGGTGGGGAGGTGCGGTCCCAGG
 wherein the coding region for said human RF-X protein begins at position 94 and ends at position 3030.

12. DNA sequence which encodes a protein according to any one of claims 3 to 8 and which hybridizes to the DNA sequence of claim 10 or 11 under conventional conditions.

13. Nucleotide sequence which is complementary to the coding strand of a DNA sequence according to any one of claims 9 to 12.

14. Fragment of a nucleotide sequence according to claim 12.

15. Fragment according to claim 14 which is the coding region of the DNA sequence of claim 11 or which is derived therefrom.

16. Recombinant DNA molecule containing a DNA sequence or nucleotide sequence according to any one of claims 9 to 15.

17. Recombinant DNA molecule according to claim 16 which is pUC / λ9 (DSM 5303) or pRF-XC (DSM 5876).

18. Recombinant DNA molecule according to claim 16 which is the expression vector pT7 / λ9 being prepared by subcloning the 3.7 kb EcoRI-insert of pUC/λ9 in phase in the expression vector pT7/7.

19. Host organism being transformed with a recombinant DNA molecule according to any of claims 16 to 18.

20. Process for the production of a protein according to any one of claims 1 to 8 comprising the steps of cultivating a transformed host organism according to claim 19 under suitable conditions in a culture medium and recovering the resulting expression product from the culture.

21. Pharmaceutical composition containing a protein according to any one of claims 1 to 8.

22. Pharmaceutical composition containing a nucleotide sequence according to claim 13 or a fragment thereof according to claim 14 or 15.

23. Pharmaceutical composition containing a protein according to any one of claims 1 to 6 or 8 for the treatment of diseases where an increase of the level of the expression of MHC class II genes is desirable.

24. Pharmaceutical composition containing a protein according to claim 7 or a corresponding recombinant protein according to claim 8 for the treatment of diseases where a decrease of the level of the expression of MHC class II genes is desirable.

25. Pharmaceutical composition containing a nucleotide sequence according to claim 13 or a fragment thereof according to claim 14 or 15 for the treatment of diseases where a decrease of the level of the expression of MHC class II genes is desirable.

26. Use of a protein according to any one of claims 1 to 8 for the screening and identification of substances capable of inhibiting the expression of MHC class II genes.

27. Pharmaceutical composition containing a protein according to any one of claims 1 to 8 or a nucleotide sequence according to claim 13 or a fragment thereof according to claim 14 or 15 or a substance which inhibits the expression of MHC class II genes identified according to claim 26 for the treatment of immune disorders.

## Claims (Claims for the following Contracting State(s): ES)

1. A method for the preparation of a protein which naturally occurs in vertebrate cells expressing MHC class II genes, regulates the expression of vertebrate MHC class II genes, and binds to the X box of DNA sequences controlling the expression of said MHC class II genes, said method comprising the culturing of a host cell transformed with a vector containing a gene encoding said protein and isolating said protein from the medium or from the cells.

2. The method according to claim 1, wherein the protein naturally occurs in mammalians, preferably in humans.

3. The method according to claim 1 or 2, wherein the protein has the biological activity of the RF-X protein which naturally occurs in human cells expressing MHC class II genes and binds to oligonucleotides containing the sequences -70 to -144 (X1) or -70 to -124 (X3) of the DRA promoter shown in Figure 4.

4. The method according to any one of claims 1 to 3 wherein the protein is the human RF-X protein.

5. The method according to claim 3 wherein the protein is a truncated form of the human RF-X protein.

6. The method according to claim 3 or 5 wherein the protein has the amino acid sequence: 
5 10 15 20 25 30
| | | | | |
1 E F G P G H V S P L Q L T N I Q V P Q Q A L P T Q R L V V Q
31 S A A P G S K G G Q V S L T V H G T Q Q V H S P P E Q S P V
61 Q A N S S S S K T A G A P T G T V P Q Q L Q V H G V Q Q S V
91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S M P M Y V S
211 G S Q V V A S S A S T G A G A S N S S G G G G S G G G G G G
241 G G G G G G G G S G S T G G G G S G A G T Y V I Q G G Y M L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V M G E F A N L A T S L N P L D P D K D E E E E E E E
781 E S E D E L P Q D I S L A A G G E S P A L G P E T L E P P A
811 K L A R T D X X G L F V Q A L P S S
 or the amino acid sequence: 
5         10         15         20         25         30
1 M A T Q A Y T E L Q A A P P P S Q P P Q A P P Q A Q P Q P P
31 P P P P P A A P Q P P Q P P T A A A T P Q P Q Y V T E L Q S
61 P Q P Q A Q P P G G Q K Q Y V T E L P A V P A P S Q P T G A
91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 wherein the amino acid sequence having 979 positions represents the entire human RF-X protein.

7. The method according to any one of claims 1 to 6 wherein the protein substantially has the DNA binding characteristics of the naturally occurring protein but is unable to activate the expression of the MHC class II genes.

8. The method according to any one of claims 1 to 7 wherein the protein is a recombinant protein.

9. A method for the preparation of a DNA sequence encoding a protein according to any one of claims 1 to 8, said method comprising the isolation of said DNA sequence from a cDNA library of recombinant bacteriophages expressing a DNA encoding said proteins by screening said library with appropriate probes.

10. The method according to claim 9 wherein the DNA sequence encodes an incomplete form of the human RF-X protein comprising the following nucleotide positions:

10        20        30        40        50
|         |         |         |         |
1 GAATTCGGGCCAGGCCACGTGTCGCCCCTCCAGCTGACCAACATCCAAGT
CTTAAGCCCGGTCCGGTGCACAGCGGGGAGGTCGACTGGTTGTAGGTTCA

51 GCCCCAGCAGGCTCTTCCCACGCAGCGTCTGGTGGTGCAGAGCGCAGCCC
CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGAGGCGGTGGCGGGGGTGGCAGTGGC
CACCACCACCGCCGCCGCCGCCCCTCCGCCACCGCCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
     GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
     GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
     CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
     GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
     CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
     TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
     GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
     CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
     CCCACTTCCACCGNNGCCGACCGCGGAAGCGCGTCTGTGACGCCGCGATG

1801 ACGTCGCTCAACCACCTGGCGCAGGCGGCGCGCTGTGCTGCAGAACAC
     TGCAGCGAGTTGGTGGACCGCGTCCGCCGCGCGACACGACGTCTTGTG

1851 CGCACAGATCAACCAGATGCTGAGCGACCTCAACCGCGTGGACTTCGCCA
     GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
     TGCACGTCCTCGTCCGGAGCACCCACACGGCGACGCTCCTGGCGCACCAC

1951 CAGCGGCTGGAGCAGGACTTCAAGGTGACGCTGCAGCAGCAGAACTCGCT
     GTCGCCGACCTCGTCCTGAAGTTCCACTGCGACGTCGTCGTCTTGAGCGA

2001 GGAGCAGTGGGCGGCCTGGCTGGACGGCGTGGTGAGCCAGGTGCTCAAGC
     CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGCGAGGAC
ACCTGGGGCTGTTTCTGCTCCTCCTTCTTCTCCTCCTCCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. The method according to claim 9 wherein the DNA sequence encodes the entire human RF-X protein comprising the following nucleotide positions: 
10 20 30 40 50
| | | | |
1 GCGAAAAGCGTTTCCGCAGACAGAAGTGGGAGAAGCGGAGGAATTAAAA
CGCTTTTCGCAAAGGCGTCTGTCTTCACCCTCTTCGCCTCCTTAATTTT

51 AAAAAAAAAAGCCTTATTTATTATCATTTTCCCCACCGTTGGCATGGCAA
TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
GTCGTCCGAGAAGGGTGCGTCGCAGACCACCACGTCTCGCGTCGGGGTCC
 
651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG

701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGAGGCGGTGGCGGGGGTGGCAGTGGCAGCA
ACCACCGCCGCCGCCGCCCCTCCGCCACCGCCCCCACCGTCACCGTCGT

1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG

1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG
 
1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1801 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCCG
GTGTCTCGAGCTGGAGGTCCCGTTCCACGACGGACTCCCGCAGCCGGGGC

1901 GGGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT

2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC

2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA
 
2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCTGTGCTGCAGAACACCGCA
GCGAGTTGGTGGACCGCGTCCGCCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG

2701 GCCAGCTTCGGTTCCTTCCACCTCATCCGGCTGCTCTACGACGAGTACAT
CGGTCGAAGCCAAGGAAGGTGGAGTAGGCCGACGAGATGCTGCTCATGTA

2751 GTACTACCTGATCGAGCACCGCGTAGCCCAGGCCAAGGGCGAGACCCCCA
CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
AGCGGCAGTACCCGCTCAAGCGGTTAGACCGGTGGAGGGACTTGGGGGAC

2851 GACCCCGACAAAGACGAGGAGGAAGAAGAGGAGGAGGAGAGCGAGGACGA
CTGGGGCTGTTTCTGCTCCTCCTTCTTCTCCTCCTCCTCTCGCTCCTGCT
 
2901 GCTGCCGCAGGACATCTCACTGGCGGCTGGCGGCGAGTCACCCGCGCTGG
CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
CCGGAGAAGCACGTCCGCGACGGGAGGTCGATTCGGGAACCGGAGGGGCG

3051 CCCACCCGCCCCCGCCACCCCTCCACGCCAGGGTCC
GGGTGGGCGGGGGCGGTGGGGAGGTGCGGTCCCAGG
 wherein the coding region for said human RF-X protein begins at position 94 and ends at position 3030.

12. A method for the preparation of a DNA sequence which encodes a protein according to any one of claims 3 to 8 and which hybridizes to the DNA sequence of claim 10 or 11 under conventional conditions, said method comprising the isolation of said DNA sequence from a cDNA library of recombinant bacteriophages expressing a DNA encoding said proteins by screening said library with appropriate probes.

13. A method for the preparation of a nucleotide sequence which is complementary to the coding strand of a DNA sequence according to any one of claims 9 to 12, said method comprising the synthesis of said sequence by enzymatic means using the DNA sequences of said claims as a template or chemical synthesis.

14. A method for the preparation of a fragment of the nucleotide sequence according to claim 12, said method comprising the enzymatic digestion of said nucleotide sequence and the isolation of the desired fragment.

15. The method according to claim 14 wherein said fragment is the coding region of the DNA sequence of claim 11 or which is derived therefrom.

16. A method for the preparation of a recombinant DNA molecule containing a DNA sequence or nucleotide sequence according to any one of claims 9 to 15, comprising the insertion of said DNA sequence or nucleotide sequence in an appropriate vector.

17. The method according to claim 16 wherein said recombinant DNA molecule is pUC/λ9 (DSM 5303) or pRF-XC (DSM 5876.)

18. A method for the preparation of the recombinant DNA molecule according to claim 16 which is the expression vector pT7/λ9, said method comprising subcloning the 3.7 kb EcoRI-insert of pUC/λ9 in phase in the expression vector pT7/7.

19. Host organism being transformed with a recombinant DNA molecule according to any of claims 16 to 18.

20. A method for the preparation of a pharmaceutical composition containing a protein prepared according to any one of claims 1 to 8, which method comprises combining said protein with a pharmaceutically acceptable carrier.

21. A method for the preparation of a pharmaceutical composition containing a nucleotide sequence prepared according to claim 13 or a fragment thereof prepared according to claim 14 or 15, which method comprises combining said nucleotide sequence or said fragment with a pharmaceutically acceptable carrier.

22. A method for the preparation of a pharmaceutical composition containing a protein prepared according to any one of claims 1 to 6 or 8 for the treatment of diseases where an increase of the level of the expression of MHC class II genes is desirable, which method comprises combining said protein with a pharmaceutically acceptable carrier.

23. A method for the preparation of a pharmaceutical composition containing a protein prepared according to claim 7 or a corresponding recombinant protein prepared according to claim 8 for the treatment of diseases where a decrease of the level of the expression of MHC class II genes is desirable, which method comprises combining said protein or said recombinant protein with a pharmaceutically acceptable carrier.

24. A method for the preparation of a pharmaceutical composition containing a nucleotide sequence prepared according to claim 13 or a fragment thereof prepared according to claim 14 or 15 for the treatment of diseases where a decrease of the level of the expression of MHC class II genes is desirable, which method comprises combining said nucleotide sequence or said fragment with a pharmaceutically acceptable carrier.

25. Use of a protein prepared according to any one of claims 1 to 8 for the screening and identification of substances capable of inhibiting the expression of MHC class II genes.

26. A method for the preparation of a pharmaceutical composition containing a protein prepared according to any one of claims 1 to 8 or a nucleotide sequence prepared according to claim 13 or a fragment thereof prepared according to claim 14 or 15 or a substance which inhibits the expression of MHC class II genes identified according to claim 25 for the treatment of immune disorders, which method comprises combining said protein, nucleotide sequence, fragment or substance with a pharmaceutically acceptable carrier.

## Patentansprüche (Patentansprüche für folgende(n) Vertragsstaat(en): AT, BE, CH, DE, DK, FR, GB, IT, LI, LU, NL, SE)

1. Protein, das natürlicherweise in Vertebratenzellen vorkommt, die MHC-Klasse II-Gene exprimieren, das die Expression von Vertebraten-MHC-Klasse II-Genen reguliert und das an die X-Box von DNA-Sequenzen bindet, die die Expression der MHC-Klasse II-Gene kontrollieren.

2. Protein nach Anspruch 1, das natürlicherweise in Säugern vorkommt, vorzugsweise in Menschen.

3. Protein nach Anspruch 1 oder 2, das die biologische Aktivität des RF-X-Proteins besitzt, das natürlicherweise in menschlichen Zellen vorkommt, die MHC-Klasse II-Gene exprimieren, und das an Oligonucleotide bindet, die die Sequenzen von -70 bis -144 (X1) oder von -70 bis -124 (X3) des in Figur 4 dargestellten DRA-Promotors enthalten.

4. Protein nach einem der Ansprüche 1 bis 3, das das menschliche RF-X-Protein ist.

5. Protein nach Anspruch 3, das eine verkürzte Form des menschlichen RF-X-Proteins ist.

6. Protein nach Anspruch 3 oder 5, das die Aminosäuresequenz 
5         10         15         20         25         30
1 E F G P G H V S P L Q L T N I Q V P Q Q A L P T Q R L V V Q
31 S A A P G S K G G Q V S L T V H G T Q Q V H S P P E Q S P V
61 Q A N S S S S K T A G A P T G T V P Q Q L Q V H G V Q Q S V
91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S H P H Y V S
211 G S Q V V A S S A S T G A G A S N S S G G G G S G G G G G G
241 G G G G G G G G S G S T G G G G S G A G T Y V I Q G G Y H L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V M G E F A N L A T S L N P L D P D K D E E E E E E E
781 E S E D E L P Q D I S L A A G G E S P A L G P E T L E P P A
811 K L A R T D X X G L F V Q A L P S S
 oder die Aminosäuresequenz 
                5         10        15        20        25        30
                |         |         |         |         |         |
  1 M A T Q A Y T E L Q A A P P P S Q P P Q A P P Q A Q P Q P P
 31 P P P P P A A P Q P P Q P P T A A A T P Q P Q Y V T E L Q S
 61 P Q P Q A Q P P G G Q K Q Y V T E L P A V P A P S Q P T G A
 91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 aufweist, wobei die Aminosäuresequenz mit 979 Resten das gesamte menschliche RF-X-Protein repräsentiert.

7. Protein nach einem der Ansprüche 1 bis 6, das im wesentlichen die DNA-Bindungseigenschaften des natürlicherweise vorkommenden Proteins aufweist, das aber nicht in der Lage ist, die Expression der MHC-Klasse II-Gene zu aktivieren.

8. Protein nach einem der Ansprüche 1 bis 7, das ein rekombinantes Protein ist.

9. DNA-Sequenz, die ein Protein nach einem der Ansprüche 1 bis 8 codiert.

10. DNA-Sequenz nach Anspruch 9, die eine unvollständige Form des menschlichen RF-X-Proteins codiert, enthaltend die folgende Nucleotidsequenz:

10        20        30        40        50
|         |         |         |         |
1 GAATTCGGGCCAGGCCACGTGTCGCCCCTCCAGCTGACCAACATCCAAGT
CTTAAGCCCGGTCCGGTGCACAGCGGGGAGGTCGACTGGTTGTAGGTTCA

51 GCCCCAGCAGGCTCTTCCCACGCAGCGTCTGGTGGTGCAGAGCGCAGCCC
CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGGAGGCGGTGGCGGGGGTGGCAGTGGC
CACCACCACCGCCGCCGCCGCCCCCTCCGCCACCGCCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
CCCACTTCCACCGNNGCCGACCGCGGAAGCGCGTCTGTGACGCCGCGATG

1801 ACGTCGCTCAACCACCTGGCGCAGGCGGCGCGCTGTGCTGCAGAACAC
TGCAGCGAGTTGGTGGACCGCGTCCGCCGCGCGACACGACGTCTTGTG

1851 CGCACAGATCAACCAGATGCTGAGCGACCTCAACCGCGTGGACTTCGCCA
GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
TGCACGTCCTCGTCCGGAGCACCCACACGGCGACGCTCCTGGCGCACCAC

1951 CAGCGGCTGGAGCAGGACTTCAAGGTGACGCTGCAGCAGAACTCGCT
GTCGCCGACCTCGTCCTGAAGTTCCACTGCGACGTCGTCTTGAGCGA

2001 GGAGCAGTGGGCGGCCTGGCTGGACGGCGTGGTGAGCCAGGTGCTCAAGC
CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGAGCGAGGAC
ACCTGGGGCTGTTTCTGCTCCTCCTTCTTCTCCTCCTCCTCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. DNA-Sequenz nach Anspruch 9, die das gesamte menschliche RF-X-Protein codiert, enthaltend die folgende Nucleotidsequenz: 
10 20 30 40 50
1 GCGAAAAGCGTTTCCGCAGACAGAAGTGGGGAGAAGCGGAGGAATTAAAA
CGCTTTTCGCAAAGGCGTCTGTCTTCACCCCTCTTCGCCTCCTTAATTTT

51 AAAAAAAAAAGCCTTATTTATTATCATTTTCCCCACCGTTGGCATGGCAA
TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
GTCGTCCGAGAAGGGTGCGTCGCAGACCACCACGTCTCGCGTCGGGGTCC

651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG
 
701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGGAGGCGGTGGCGGGGGTGGCAGTGGCAGCA
ACCACCGCCGCCGCCGCCCCCTCCGCCACCGCCCCCACCGTCACCGTCGT

1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG

1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG
 
1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1801 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCG
GTGTCTCGAGCTGGAGGTCCCGTTCCACGACGGACTCCCGCAGCCGGGC

1901 GGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT

2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC

2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA
 
2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCTGTGCTGCAGAACACCGCA
GCGAGTTGGTGGACCGCGTCCGCCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG

2701 GCCAGCTTCGGTTCCTTCCACCTCATCCGGCTGCTCTACGACGAGTACAT
CGGTCGAAGCCAAGGAAGGTGGAGTAGGCCGACGAGATGCTGCTCATGTA

2751 GTACTACCTGATCGAGCACCGCGTAGCCCAGGCCAAGGGCGAGACCCCCA
CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
AGCGGCAGTACCCGCTCAAGCGGTTAGACCGGTGGAGGGACTTGGGGGAC
 
2851 GACCCCGACAAAGACGAGGAGGAAGAAGAGGAGGAGGAGAGCGAGGACGA
CTGGGGCTGTTTCTGCTCCTCCTTCTTCTCCTCCTCCTCTCGCTCCTGCT

2901 GCTGCCGCAGGACATCTCACTGGCGGCTGGCGGCGAGTCACCCGCGCTGG
CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
CCGGAGAAGCACGTCCGCGACGGGAGGTCGATTCGGGAACCGGAGGGGCG

3051 CCCACCCGCCCCCGCCACCCCTCCACGCCAGGGTCC
GGGTGGGCGGGGGCGGTGGGGAGGTGCGGTCCCAGG
 wobei der codierende Bereich für das menschliche RF-X-Protein bei Position 94 beginnt und bei Position 3030 endet.

12. DNA-Sequenz, die ein Protein nach einem der Ansprüche 3 bis 8 codiert und die unter konventionellen Bedingungen mit der DNA-Sequenz nach Anspruch 10 oder 11 hybridisiert.

13. Nucleotidsequenz, die komplementär ist zu dem codierenden Strang einer DNA-Sequenz nach einem der Ansprüche 9 bis 12.

14. Fragment einer Nucleotidsequenz nach Anspruch 12.

15. Fragment nach Anspruch 14, das der codierende Bereich der DNA-Sequenz nach Anspruch 11 ist oder das von diesem abgeleitet ist.

16. Rekombinantes DNA-Molekül, enthaltend eine DNA-Sequenz oder eine Nucleotidsequenz nach einem der Ansprüche 9 bis 15.

17. Rekombinantes DNA-Molekül nach Anspruch 16, das pUC/λ9 (DSM 5303) oder pRF-XC (DSM 5876) ist.

18. Rekombinantes DNA-Molekül nach Anspruch 16, das der Expressionsvektor pT7/λ9 ist, hergestellt durch die in Phase-Subclonierung der 3,7 kb großen EcoRI-Insertion von pUC/λ9 in den Expressionsvektor pT7/7.

19. Wirtsorganismus, der mit einem rekombinanten DNA-Molekül nach einem der Ansprüche 16 bis 18 transformiert ist.

20. Verfahren zur Herstellung eines Proteins nach einem der Ansprüche 1 bis 8, umfassend die Kultivierung eines transformierten Wirtsorganismus nach Anspruch 19 unter geeigneten Bedingungen in einem Kulturmedium und Gewinnung des resultierenden Expressionsproduktes aus der Kultur.

21. Arzneimittel, enthaltend ein Protein nach einem der Ansprüche 1 bis 8.

22. Arzneimittel, enthaltend eine Nucleotidsequenz nach Anspruch 13 oder ein Fragment davon nach Anspruch 14 oder 15.

23. Arzneimittel, enthaltend ein Protein nach einem der Ansprüche 1 bis 6 oder 8 zur Behandlung von Krankheiten, bei denen eine Steigerung der Expressionsrate von MHC-Klasse II-Genen wünschenswert ist.

24. Arzneimittel, enthaltend ein Protein nach Anspruch 7 oder ein entsprechendes rekombinantes Protein nach Anspruch 8 zur Behandlung von Krankheiten, bei denen eine Verringerung der Expressionsrate von MHC-Klasse II-Genen wünschenswert ist.

25. Arzneimittel, enthaltend eine Nucleotidsequenz nach Anspruch 13 oder ein Fragment davon nach Anspruch 14 oder 15 zur Behandlung von Krankheiten, bei denen eine Verringerung der Expressionsrate von MHC-Klasse II-Genen wünschenswert ist.

26. Verwendung eines Proteins nach einem der Ansprüche 1 bis 8 zum Durchmustern und Identifizieren von Substanzen, die in der Lage sind, die Expression von MHC-Klasse II-Genen zu hemmen.

27. Arzneimittel, enthaltend ein Protein nach einem der Ansprüche 1 bis 8 oder eine Nucleotidsequenz nach Anspruch 13 oder ein Fragment davon nach Anspruch 14 oder 15 oder eine Substanz, die die Expression von MHC-Klasse II-Genen hemmt und die nach Anspruch 26 identifiziert wurde, zur Behandlung von Immunstörungen.

## Patentansprüche (Patentansprüche für folgende(n) Vertragsstaat(en): ES)

1. Verfahren zur Herstellung eines Proteins, das natürlicherweise in Vertebratenzellen vorkommt, die MHC-Klasse II-Gene exprimieren, das die Expression von Vertebraten-MHC-Klasse II-Genen reguliert und das an die X-Box von DNA-Sequenzen bindet, die die Expression der MHC-Klasse II-Genen kontrollieren, wobei das Verfahren die Kultivierung einer Wirtszelle umfaßt, die mit einem Vektor transformiert ist, der ein das Protein codierendes Gen enthält, und die Isolierung des Proteins aus dem Medium oder den Zellen.

2. Verfahren nach Anspruch 1, wobei das Protein natürlicherweise in Säugern vorkommt, vorzugsweise in Menschen.

3. Verfahren nach Anspruch 1 oder 2, wobei das Protein die biologische Aktivität des RF-X-Proteins besitzt, das natürlicherweise in menschlichen Zellen vorkommt, die MHC-Klasse II-Gene exprimieren, und das an Oligonucleotide bindet, die die Sequenzen von -70 bis -144 (X1) oder von -70 bis -124 (X3) des in Figur 4 dargestellten DRA-Promotors enthalten.

4. Verfahren nach einem der Ansprüche 1 bis 3, wobei das Protein das menschliche RF-X-Protein ist.

5. Verfahren nach Anspruch 3, wobei das Protein eine verkürzte Form des menschlichen RF-X-Proteins ist.

6. Verfahren nach Anspruch 3 oder 5, wobei das Protein die Aminosäuresequenz 
5 10 15 20 25 30
1 E F G P G H V S P L Q L T N I Q V P Q Q A L P T Q R L V V Q
31 S A A P G S K G G Q V S L T V H G T Q Q V H S P P E Q S P V
61 Q A N S S S S K T A G A P T G T V P Q Q L Q V H G V Q Q S V
91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S H P H Y V S
211 G S Q V V A S S A S T G A G A S N S S G G G G S G G G G G G
241 G G G G G G G G S G S T G G G G S G A G T Y V I Q G G Y M L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V M G E F A N L A T S L N P L D P D K D E E E E E E E
781 E S E D E L P Q D I S L A A G G E S P A L G P E T L E P P A
811 K L A R T D X X G L F V Q A L P S S
 oder die Aminosäuresequenz 
5 10 15 20 25 30
| | | | | |
1 M A T Q A Y T E L Q A A P P P S Q P P Q A P P Q A Q P Q P P
31 P P P P P A A P Q P P Q P P T A A A T P Q P Q Y V T E L Q S
61 P Q P Q A Q P P G G Q K Q Y V T E L P A V P A P S Q P T G A
91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 aufweist, wobei die Aminosäuresequenz mit 979 Resten das gesamte menschliche RF-X-Protein repräsentiert.

7. Verfahren nach einem der Ansprüche 1 bis 6, wobei das Protein im wesentlichen die DNA-Bindungseigenschaften des natürlicherweise vorkommenden Proteins besitzt, aber nicht in der Lage ist, die Expression der MHC-Klasse II-Gene zu aktivieren.

8. Verfahren nach einem der Ansprüche 1 bis 7, wobei das Protein ein rekombinantes Protein ist.

9. Verfahren zur Herstellung einer DNA-Sequenz, die ein Protein nach einem der Ansprüche 1 bis 8 codiert, umfassend die Isolierung der DNA-Sequenz aus einer cDNA-Bibliothek rekombinanter Bakteriophagen exprimierend eine DNA, die die Proteine codiert, durch Durchmusterung der Bibliothek mit geeigneten Sonden.

10. Verfahren nach Anspruch 9, wobei die DNA-Sequenz eine unvollständige Form des menschlichen RF-X-Proteins codiert und die folgende Nucleotidsequenz enthält:

10        20        30        40        50
1 GAATTCGGGCCAGGCCACGTGTCGCCCCTCCAGCTGACCAACATCCAAGT
CTTAAGCCCGGTCCGGTGCACAGCGGGGAGGTCGACTGGTTGTAGGTTCA

51 GCCCCAGCAGGCTCTTCCCACGCAGCGTCTGGTGGTGCAGAGCGCAGCCC
CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGGAGGCGGTGGCGGGGGTGGCAGTGGC
CACCACCACCGCCGCCGCCGCCCCCTCCGCCACCGCCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
     GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
     GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
     CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
     GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
     CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
     TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
     GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
     CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
     CCCACTTCCACCGNNGCCGACCGCGGAAGCGCGTCTGTGACGCCGCGATG

1801 ACGTCGCTCAACCACCTGGCGCAGGCGGCGCGCGCTGTGCTGCAGAACAC
     TGCAGCGAGTTGGTGGACCGCGTCCGCCGCGCGCGACACGACGTCTTGTG

1851 CGCACAGATCAACCAGATGCTGAGCGACCTCAACCGCGTGGACTTCGCCA
     GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
     TGCACGTCCTCGTCCGGAGCACCCACACGGCGACGCTCCTGGCGCACCAC

1951 CAGCGGCTGGAGCAGGACTTCAAGGTGACGCTGCAGCAGCAGAACTCGCT
     GTCGCCGACCTCGTCCTGAAGTTCCACTGCGACGTCGTCGTCTTGAGCGA

2001 GGAGCAGTGGGCGGCCTGGCTGGACGGCGTGGTGAGCCAGGTGCTCAAGC
     CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGAGCGAGGAC
ACCTGGGGCTGTTTCTGCTCCTCCTTCTTCTCCTCCTCCTCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. Verfahren nach Anspruch 9, wobei die DNA-Sequenz das gesamte menschliche RF-X-Protein codiert und die folgende Nucleotidsequenz enthält: 
10        20        30        40        50
1 GCGAAAAGCGTTTCCGCAGACAGAAGTGGGGAGAAGCGGAGGAATTAAAA
  CGCTTTTCGCAAAGGCGTCTGTCTTCACCCCTCTTCGCCTCCTTAATTTT

51 AAAAAAAAAAGCCTTATTTATTATCATTTTCCCCACCGTTGGCATGGCAA
   TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
    GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
    GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
    CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
    TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
    CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
    CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
    AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
    TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
    CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
    GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
    GTCGTCCGAGAAGGGTGCGTCGCAGACCACCACGTCTCGCGTCGGGGTCC
 
651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG

701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGGAGGCGGTGGCGGGGGTGGCAGTGGCAGCA
ACCACCGCCGCCGCCGCCCCCTCCGCCACCGCCCCCACCGTCACCGTCGT

1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG

1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG
 
1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1801 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCG
GTGTCTCGAGCTGGAGGTCCCGTTCCACGACGGACTCCCGCAGCCGGGC

1901 GGGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT

2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC

2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA
 
2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCTGTGCTGCAGAACACCGCA
GCGAGTTGGTGGACCGCGTCCGCCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG

2701 GCCAGCTTCGGTTCCTTCCACCTCATCCGGCTGCTCTACGACGAGTACAT
CGGTCGAAGCCAAGGAAGGTGGAGTAGGCCGACGAGATGCTGCTCATGTA

2751 GTACTACCTGATCGAGCACCGCGTAGCCCAGGCCAAGGGCGAGACCCCCA
CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
AGCGGCAGTACCCGCTCAAGCGGTTAGACCGGTGGAGGGACTTGGGGGAC
 
2851 GACCCCGACAAAGACGAGGAGGAAGAAGAGGAGGAGGAGAGCGAGGACGA
CTGGGGCTGTTTCTGCTCCTCCTTCTTCTCCTCCTCCTCTCGCTCCTGCT

2901 GCTGCCGCAGGACATCTCACTGGCGGCTGGCGGCGAGTCACCCGCGCTGG
CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
CCGGAGAAGCACGTCCGCGACGGGAGGTCGATTCGGGAACCGGAGGGGCG

3051 CCCACCCGCCCCCGCCACCCCTCCACGCCAGGTCC
GGGTGGGCGGGGGCGGTGGGGAGGTGCGGTCCAGG
 wobei der codierende Bereich für das menschliche RF-X-Protein bei Position 94 beginnt und bei Position 3030 endet.

12. Verfahren zur Herstellung einer DNA-Sequenz, die ein Protein nach einem der Ansprüche 3 bis 8 codiert und die unter konventionellen Bedingungen mit einer DNA-Sequenz nach Anspruch 10 oder 11 hybridisiert, umfassend die Isolierung der DNA-Sequenz aus einer cDNA-Bibliothek rekombinanter Phagen, die eine die Proteine codierende DNA exprimieren, durch Durchmusterung der Bibliothek mit geeigneten Sonden.

13. Verfahren zur Herstellung einer Nucleotidsequenz, die komplementär ist zu dem codierenden Strang einer DNA-Sequenz nach einem der Ansprüche 9 bis 12, umfassend die Synthese der Sequenz mit Hilfe von Enzymen unter Verwendung der DNA-Sequenzen der bezeichneten Ansprüche als Matrizen oder durch chemische Synthese.

14. Verfahren zur Herstellung eines Fragmentes der Nucleotidsequenz nach Anspruch 12, umfassend die enzymatische Spaltung der Nucleotidsequenz und die Isolierung des gewünschten Fragmentes.

15. Verfahren nach Anspruch 14, wobei das Fragment der codierende Bereich der DNA-Sequenz nach Anspruch 11 ist oder von dieser abgeleitet ist.

16. Verfahren zur Herstellung eines rekombinanten DNA-Moleküls, das eine DNA-Sequenz oder eine Nucleotidsequenz nach einem der Ansprüche 9 bis 15 enthält, umfassend die Insertion der DNA-Sequenz oder Nucleotidsequenz in einen geeigneten Vektor.

17. Verfahren nach Anspruch 16, wobei das rekombinante DNA-Molekül pUC/λ9 (DSM 5303) oder pRF-XC (DSM 5876) ist.

18. Verfahren zur Herstellung des rekombinanten DNA-Moleküls nach Anspruch 16, das der Expressionsvektor pT7/λ9 ist, umfassend die in Phase-Subclonierung der 3,7 kb großen EcoRI-Insertion von pUC/λ9 in den Expressionsvektor pT7/7.

19. Wirtsorganismus, der mit einem rekombinanten DNA-Molekül nach einem der Ansprüche 16 bis 18 transformiert ist.

20. Verfahren zur Herstellung eines Arzneimittels, enthaltend ein Protein, das gemäß einem der Ansprüche 1 bis 8 hergestellt wurde, wobei das Protein mit einem pharmazeutisch verträglichen Trägerstoff kombiniert wird.

21. Verfahren zur Herstellung eines Arzneimittels, enthaltend eine Nucleotidsequenz, die gemäß Anspruch 13 hergestellt wurde, oder ein Fragment davon, das gemäß Anspruch 14 oder 15 hergestellt wurde, wobei die Nucleotidsequenz oder das Fragment mit einem pharmazeutisch verträglichen Trägerstoff kombiniert wird.

22. Verfahren zur Herstellung eines Arzneimittels enthaltend ein Protein, das gemäß einem der Ansprüche 1 bis 6 oder 8 hergestellt wurde, zur Behandlung von Krankheiten, bei denen eine Steigerung der Expressionsrate von MHC-Klasse II-Genen wünschenswert ist, wobei das Protein mit einem pharmazeutisch verträglichen Trägerstoff kombiniert wird.

23. Verfahren zur Herstellung eines Arzneimittels enthaltend ein Protein, das gemäß Anspruch 7 hergestellt wurde, oder ein entsprechendes rekombinantes Protein, das gemäß Anspruch 8 hergestellt wurde, zur Behandlung von Krankheiten, bei denen eine Verringerung der Expressionsrate von MHC-Klasse II-Genen wünschenswert ist, wobei das Protein oder das rekombinante Protein mit einem pharmazeutisch verträglichen Trägerstoff kombiniert wird.

24. Verfahren zur Herstellung eines Arzneimittels, enthaltend eine Nucleotidsequenz, die gemäß Anspruch 13 hergestellt wurde, oder ein Fragment davon, das gemäß Anspruch 14 oder 15 hergestellt wurde, zur Behandlung von Krankheiten, bei denen eine Verringerung der Expressionsrate von MHC-Klasse II-Genen wünschenswert ist, wobei die Nucleotidsequenz oder das Fragment mit einem pharmazeutisch verträglichen Trägerstoff kombiniert wird.

25. Verwendung eines Proteins, das gemäß einem der Ansprüche 1 bis 8 hergestellt wurde, zum Durchmustern und Identifizieren von Substanzen, die in der Lage sind, die Expression von MHC-Klasse II Genen zu hemmen.

26. Verfahren zur Herstellung eines Arzneimittels enthaltend ein Protein, das gemäß einem der Ansprüche 1 bis 8 hergestellt wurde, oder eine Nucleotidsequenz, die gemäß Anspruch 13 hergestellt wurde, oder ein Fragment davon, das gemäß Anspruch 14 oder 15 hergestellt wurde, oder eine Substanz, die die Expression von MHC-Klasse II-Genen hemmt und gemäß Anspruch 25 identifiziert wurde, zur Behandlung von Immunstörungen, wobei das Protein, die Nucleotidsequenz, das Fragment oder die Substanz mit einem pharmazeutisch verträglichen Trägerstoff kombiniert wird.

## Revendications (Revendications pour l'(les) Etat(s) contractant(s) suivant(s): AT, BE, CH, DE, DK, FR, GB, IT, LI, LU, NL, SE)

1. Protéine qui existe naturellement dans les cellules de vertébrés exprimant les gènes CMH de classe II, qui régule l'expression des gènes CMH de classe II des vertébrés et qui se lie à la boîte X des séquences d'ADN contrôlant l'expression desdits gènes CMH de classe II.

2. Protéine selon la revendication 1 qui existe naturellement chez les mammifères, de préférence chez l'homme.

3. Protéine selon la revendication 1 ou 2 qui a l'activité biologique de la protéine RF-X qui existe naturellement dans les cellules humaines exprimant les gènes CMH de classe II et qui se lie aux oligonucléotides contenant les séquences -70 à -144 (X1) ou -70 à -124 (X3) du promoteur DRA montré sur la figure 4.

4. Protéine selon l'une quelconque des revendications 1 à 3 qui est la protéine RF-X humaine.

5. Protéine selon la revendication 3 qui est une forme tronquée de la protéine RF-X humaine.

6. Protéine selon la revendication 3 ou 5 qui a la séquence d'acides aminés : 
5         10        15        20        25        30
|          |          |          |          |          |
1 E F G P G H V S P L Q L T N I Q V P Q Q A L P T Q R L V V Q
31 S A A P G S K G G Q V S L T V H G T Q Q V H S P P E Q S P V
61 Q A N S S S S K T A G A P T G T V P Q Q L Q V H G V Q Q S V
91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S M P M Y V S
211 G S Q V V A S S A S T G A G A S N S S G G G G S G G G G G G
241 G G G G G G G G S G S T G G G G S G A G T Y V I Q G G Y M L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V M G E F A N L A T S L N P L D P D K D E E E E E E E
781 E S E D E L P Q D I S L A A G G E S P A L G P E T L E P P A
611 K L A R T D X X G L F V Q A L P S S
 ou la séquence d'acides aminés : 
5 10 15 20 25 30
1 M A T Q A Y T E L Q A A P P P S Q P P Q A P P Q A Q P Q P P
31 P P P P P A A P Q P P Q P P T A A A T P Q P Q Y V T E L Q S
61 P Q P Q A Q P P G G Q K Q Y V T E L P A V P A P S Q P T G A
91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 où la séquence d'acides aminés ayant 979 positions représente la protéine RF-X humaine totale.

7. Protéine selon l'une quelconque des revendications 1 à 6 qui a sensiblement les caractéristiques de liaison à l'ADN de la protéine naturelle mais qui est incapable d'activer l'expression des gènes CMH de classe II.

8. Protéine selon l'une quelconque des revendications 1 à 7 qui est une protéine recombinée.

9. Séquence d'ADN codant une protéine selon l'une quelconque des revendications 1 à 8.

10. Séquence d'ADN selon la revendication 9 qui code une forme incomplète de la protéine RF-X humaine comprenant les positions de nucléotides suivantes :

10 20 30 40 50
| | | | |
1 GAATTCGGGCCAGGCCACGTGTCGCCCCTCCAGCTGACCAACATCCAAGT
CTTAAGCCCGGTCCGGTGCACAGCGGGGAGGTCGACTGGTTGTAGGTTCA

51 GCCCCAGCAGGCTCTTCCCACGCAGCGTCTGGTGGTGCAGAGCGCAGCCC
CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGGAGGCGGTGGCGGGGGTGGCAGTGGC
CACCACCACCGCCGCCGCCGCCCCCTCCGCCACCGCCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
CCCACTTCCACCGNNGCCGACCGCGGAAGCGCGTCTGTGACGCCGCGATG

1801 ACGTCGCTCAACCACCTGGCGCAGGCGGCGCGCTGTGCTGCAGAACAC
TGCAGCGAGTTGGTGGACCGCGTCCGCCGCGCGACACGACGTCTTGTG

1851 CGCACAGATCAACCAGATGCTGAGCGACCTCAACCGCGTGGACTTCGCCA
GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
TGCACGTCCTCGTCCGGAGCACCCACACGGCGACGCTCCTGGCGCACCAC

1951 CAGCGGCTGGAGCAGGACTTCAAGGTGACGCTGCAGCAGCAGAACTCGCT
GTCGCCGACCTCGTCCTGAAGTTCCACTGCGACGTCGTCGTCTTGAGCGA

2001 GGAGCAGTGGGCGGCCTGGCTGGACGGCGTGGTGAGCCAGGTGCTCAAGC
CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGAGCGAGGAC
ACCTGGGGCTGTTTCTGCTCCTCCTTCTTCTCCTCCTCCTCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. Séquence d'ADN selon la revendication 9 qui code la protéine RF-X humaine entière comprenant les positions de nucléotides suivantes : 
10 20 30 40 50
1 GCGAAAAGCGTTTCCGCAGACAGAAGTGGGGAGAAGCGGAGGAATTAAAA
CGCTTTTCGCAAAGGCGTCTGTCTTCACCCCTCTTCGCCTCCTTAATTTT

51 AAAAAAAAAAGCCTTATTTATTATCATTTTCCCCACCGTTGGCATGGCAA
TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
GTCGTCCGAGAAGGGTGCGTCGCAGACCACCACGTCTCGCGTCGGGGTCC
 
651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG

701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGAGGCGGTGGCGGGGGTGGCAGTGGCAGCA
ACCACCGCCGCCGCCGCCCCTCCGCCACCGCCCCCACCGTCACCGTCGT

1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG
 
1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG

1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1801 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCG
GTGTCTCGAGCTGGAGGTCCCGTTCCACGACGGACTCCCGCAGCCCGGGC

1901 GGGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT

2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC
 
2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA

2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCTGTGCTGCAGAACACCGCA
GCGAGTTGGTGGACCGCGTCCGCCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG

2701 GCCAGCTTCGGTTCCTTCCACCTCATCCGGCTGCTCTACGACGAGTACAT
CGGTCGAAGCCAAGGAAGGTGGAGTAGGCCGACGAGATGCTGCTCATGTA
 
2751 GTACTACCTGATCGAGCACCGCGTAGCCCAGGCCAAGGGCGAGACCCCCA
CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
AGCGGCAGTACCCGCTCAAGCGGTTAGACCGGTGGAGGGACTTGGGGGAC

2851 GACCCCGACAAAGACGAGGAGGAAGAAGAGGAGGAGGAGAGCGAGGACGA
CTGGGGCTGTTTCTGCTCCTCCTTCTTCTCCTCCTCCTCTCGCTCCTGCT

2901 GCTGCCGCAGGACATCTCACTGGCGGCTGGCGGCGAGTCACCCGCGCTGG
CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
CCGGAGAAGCACGTCCGCGACGGGAGGTCGATTCGGGAACCGGAGGGGCG

3051 CCCACCCGCCCCCGCCACCCCTCCACGCCAGGGTCC
GGGTGGGCGGGGGCGGTGGGGAGGTGCGGTCCCAGG
 où la région codante pour ladite protéine RF-X humaine commence en position 94 et se termine en position 3030.

12. Séquence d'ADN qui code une protéine selon l'une quelconque des revendications 3 à 8 et qui s'hybride avec la séquence d'ADN de la revendication 10 ou 11 dans des conditions conventionnelles.

13. Séquence nucléotidique qui est complémentaire du brin codant d'une séquence d'ADN selon l'une quelconque des revendications 9 à 12.

14. Fragment d'une séquence nucléotidique selon la revendication 12.

15. Fragment selon la revendication 14 qui est la région codante de la séquence d'ADN de la revendication 11 ou qui en est dérivé.

16. Molécule d'ADN recombiné contenant une séquence d'ADN ou une séquence nucléotidique selon l'une quelconque des revendications 9 à 15.

17. Molécule d'ADN recombiné selon la revendication 16 qui est pUC/λ9 (DSM 5303) ou pRF-XC (DSM 5876).

18. Molécule d'ADN recombiné selon la revendication 16 qui est le vecteur d'expression pT7/λ9 préparée par sous-clonage de l'insert EcoRI de 3,7 kb de pUC/λ9 en phase dans le vecteur d'expression pT7/7.

19. Organisme hôte transformé avec une molécule d'ADN recombiné selon l'une quelconque des revendications 16 à 18.

20. Procédé de production d'une protéine selon l'une quelconque des revendications 1 à 8 qui comprend les étapes de culture d'un organisme hôte transformé selon la revendication 19 dans des conditions appropriées dans un milieu de culture et de récupération du produit d'expression résultant à partir de la culture.

21. Composition pharmaceutique contenant une protéine selon l'une quelconque des revendications 1 à 8.

22. Composition pharmaceutique contenant une séquence nucléotidique selon la revendication 13 ou un fragment de celle-ci selon la revendication 14 ou 15.

23. Composition pharmaceutique contenant une protéine selon l'une quelconque des revendications 1 à 6 ou 8 pour le traitement des maladies dans lesquelles une augmentation du niveau d'expression des gènes CMH de classe II est souhaitable.

24. Composition pharmaceutique contenant une protéine selon la revendication 7 ou une protéine recombinée correspondante selon la revendication 8 pour le traitement des maladies dans lesquelles une diminution du niveau de l'expression des gènes CMH de classe II est souhaitable.

25. Composition pharmaceutique contenant une séquence nucléotidique selon la revendication 13 ou un fragment de celle-ci selon la revendication 14 ou 15 pour le traitement des maladies dans lesquelles une diminution du niveau de l'expression des gènes CMH de classe II est souhaitable.

26. Utilisation d'une protéine selon l'une quelconque des revendications 1 à 8 pour le criblage et l'identification de substances capables d'inhiber l'expression des gènes CMH de classe II.

27. Composition pharmaceutique contenant une protéine selon l'une quelconque des revendications 1 à 8 ou une séquence nucléotidique selon la revendication 13 ou un fragment de celle-ci selon la revendication 14 ou 15 ou une substance qui inhibe l'expression des gènes CMH de classe II identifiée selon la revendication 26 pour le traitement des troubles immuns.

## Revendications (Revendications pour l'(les) Etat(s) contractant(s) suivant(s): ES)

1. Procédé de préparation d'une protéine qui existe naturellement dans les cellules de vertébrés exprimant les gènes CMH de classe II, qui régule l'expression des gènes CMH de classe II des vertébrés et qui se lie à la boîte X des séquences d'ADN contrôlant l'expression desdits gènes CMH de classe II, ledit procédé comprenant la culture d'une cellule hôte transformée avec un vecteur contenant un gène codant ladite protéine et l'isolement de ladite protéine à partir du milieu ou à partir des cellules.

2. Procédé selon la revendication 1 dans lequel la protéine existe naturellement chez les mammifères, de préférence chez l'homme.

3. Procédé selon la revendication 1 ou 2 dans lequel la protéine a l'activité biologique de la protéine RF-X qui existe naturellement dans les cellules humaines exprimant les gènes CMH de classe II et se lie aux oligonucléotides contenant les séquences -70 à -144 (X1) ou -70 à -124 (X3) du promoteur DRA montré sur la figure 4.

4. Procédé selon l'une quelconque des revendications 1 à 3 dans lequel la protéine est la protéine RF-X humaine.

5. Procédé selon la revendication 3 dans lequel la protéine est une forme tronquée de la protéine RF-X humaine.

6. Procédé selon la revendication 3 ou 5 dans lequel la protéine a la séquence d'acides aminés : 
                5         10        15        20        25        30
                |         |         |         |         |         |
  1 E F G P G H V S P L Q L T N I Q V P Q Q A L P T Q R L V V Q
 31 S A A P G S K G G Q V S L T V H G T Q Q V H S P P E Q S P V
 61 Q A N S S S S K T A G A P T G T V P Q Q L Q V H G V Q Q S V
 91 P V T Q E R S V V Q A T P Q A P K P G P V Q P L T V Q G L Q
121 P V H V A Q E V Q Q L Q Q V P V P H V Y S S Q V Q Y V E G G
151 D A S Y T A S A I R S S T Y S Y P E T P L Y T Q T A S T S Y
181 Y E A A G T A T Q V S T P A T S Q A V A S S G S M P M Y V S
211 G S Q V V A S S A S T G A G A S N S S G G G G S G G G G G G
241 G G G G G G G G S G S T G G G G S G A G T Y V I Q G G Y M L
271 G S A S Q S Y S H T T R A S P A T V Q W L L D N Y E T A E G
301 V S L P R S T L Y C H Y L L H C Q E Q K L E P V N A A S F G
331 K L I R S V F M G L R T R R L G T R G N S K Y H Y Y G L R I
361 K A S S P L L R L M E D Q Q H M A M R G Q P F S Q K Q R L K
391 P I Q K M E G M T N G V A V G Q Q P S T G L S D I S A Q V Q
421 Q Y Q Q F L D A S R S L P D F T E L D L Q G K V L P E G V G
451 P G D I K A F Q V L Y R E H C E A I V D V M V N L Q F T L V
481 E T L W K T F W R Y N L S Q P S E A P P L A V H D E A E K R
511 L P K A I L V L L S K F E P V L Q W T K H C D N V L Y Q G L
541 V E I L I P D V L R P I P S A L T Q A I R N F A K S L E S W
571 L T H A M V N I P E E M L R V K V X A A G A F A Q T L R R Y
601 T S L N H L A Q A A R A V L Q N T A Q I N Q M L S D L N R V
631 D F A N V Q E Q A S W V C R C E D R V V Q R L E Q D F K V T
661 L Q Q Q N S L E Q W A A W L D G V V S Q V L K P Y Q G S A G
691 F P K A A K L F L L K W S F Y S S M V I R D L T L R T A A S
721 F G S F H L I R L L Y D E Y M Y Y L I E H R V A Q A K G E T
751 P I A V H G E F A N L A T S L N P L D P D K D E E E E E E E
781 E S E D E L P Q D I S L A A G G E S P A L G P E T L E P P A
811 K L A R T D X K G L F V Q A L P S S
 ou la séquence d'acides aminés : 
5 10 15 20 25 30
1 M A T Q A Y T E L Q A A P P P S Q P P Q A P P Q A Q P Q P P
31 P P P P P A A P Q P P Q P P T A A A T P Q P Q Y V T E L Q S
61 P Q P Q A Q P P G G Q K Q Y V T E L P A V P A P S Q P T G A
91 P T P S P A P Q Q Y I V V T V S E G A M R A S E T V S E A S
121 P G S T A S Q T G V P T Q V V Q Q V Q G T Q Q R L L V Q T S
151 V Q A K P G H V S P L Q L T N I Q V P Q Q A L P T Q R L V V
181 Q S A A P G S K G G Q V S L T V H G T Q Q V H S P P E Q S P
211 V Q A N S S S S K T A G A P T G T V P Q Q L Q V H G V Q Q S
241 V P V T Q E R S V V Q A T P Q A P K P G P V Q P L T V Q G L
271 Q P V H V A Q E V Q Q L Q Q V P V P H V Y S S Q V Q Y V E G
301 G D A S Y T A S A I R S S T Y S Y P E T P L Y T Q T A S T S
331 Y Y E A A G T A T Q V S T P A T S Q A V A S S G S M P M Y V
361 S G S Q V V A S S A S T G A G A S N S S G G G G S G G G G G
391 G G G G G G G G G S G S T G G G G S G A G T Y V I Q G G Y M
421 L G S A S Q S Y S H T T R A S P A T V Q W L L D N Y E T A E
451 G V S L P R S T L Y C H Y L L H C Q E Q K L E P V N A A S F
481 G K L I R S V F M G L R T R R L G T R G N S K Y H Y Y G L R
511 I K A S S P L L R L M E D Q Q H M A M R G Q P F S Q K Q R L
541 K P I Q K M E G M T N G V A V G Q Q P S T G L S D I S A Q V
571 Q Q Y Q Q F L D A S R S L P D F T E L D L Q G K V L P E G V
601 G P G D I K A F Q V L Y R E H C E A I V D V M V N L Q F T L
631 V E T L W K T F W R Y N L S Q P S E A P P L A V H D E A E K
661 R L P K A I L V L L S K F E P V L Q W T K H C D N V L Y Q G
691 L V E I L I P D V L R P I P S A L T Q A I R N F A K S L E S
721 W L T H A M V N I P E E M L R V K V A A A G A F A Q T L R R
751 Y T S L N H L A Q A A R A V L Q N T A Q I N Q M L S D L N R
781 V D F A N V Q E Q A S W V C R C E D R V V Q R L E Q D F K V
811 T L Q Q Q N S L E Q W A A W L D G V V S Q V L K P Y Q G S A
841 G F P K A A K L F L L K W S F Y S S M V I R D L T L R S A A
871 S F G S F H L I R L L Y D E Y M Y Y L I E H R V A Q A K G E
901 T P I A V M G E F A N L A T S L N P L D P D K D E E E E E E
931 E E S E D E L P Q D I S L A A G G E S P A L G P E T L E P P
961 A K L A R T D A R G L F V Q A L P S S
 où la séquence d'acides aminés ayant 979 positions représente la protéine RF-X humaine entière.

7. Procédé selon l'une quelconque des revendications 1 à 6 dans lequel la protéine a sensiblement les caractéristiques de liaison à l'ADN de la protéine naturelle mais est incapable d'activer l'expression des gènes CMH de classe II.

8. Procédé selon l'une quelconque des revendications 1 à 7 dans lequel la protéine est une protéine recombinée.

9. Procédé de préparation d'une séquence d'ADN codant une protéine selon l'une quelconque des revendications 1 à 8, ledit procédé comprenant l'isolement de ladite séquence d'ADN à partir d'une banque d'ADNc de bactériophages recombinés exprimant un ADN codant lesdites protéines par criblage de ladite banque avec des sondes appropriées.

10. Procédé selon la revendication 9, dans lequel la séquence d'ADN code une forme incomplète de la protéine RF-X humaine comprenant les positions de nucléotides suivantes :

10        20        30        40        50
1 GAATTCGGGCCAGGCCACGTGTCGCCCCTCCAGCTGACCAACATCCAAGT
CTTAAGCCCGGTCCGGTGCACAGCGGGGAGGTCGACTGGTTGTAGGTTCA

51 GCCCCAGCAGGCTCTTCCCACGCAGCGTCTGGTGGTGCAGAGCGCAGCCC
CGGGGTCGTCCGAGAAGGGTGCGTCGCAGACCACCACGTCTCGCGTCGGG

101 CAGGCAGCAAAGGTGGCCAGGTCTCCCTGACGGTCCATGGTACCCAGCAG
GTCCGTCGTTTCCACCGGTCCAGAGGGACTGCCAGGTACCATGGGTCGTC

151 GTGCACTCGCCCCCAGAGCAGTCGCCGGTGCAGGCCAACAGCTCTTCCAG
CACGTGAGCGGGGGTCTCGTCAGCGGCCACGTCCGGTTGTCGAGAAGGTC

201 CAAGACAGCCGGGGCCCCCACGGGCACAGTGCCACAGCAGCTGCAGGTCC
GTTCTGTCGGCCCCGGGGGTGCCCGTGTCACGGTGTCGTCGACGTCCAGG

251 ACGGCGTCCAGCAGAGTGTCCCCGTCACCCAAGAGAGATCTGTGGTCCAG
TGCCGCAGGTCGTCTCACAGGGGCAGTGGGTTCTCTCTAGACACCAGGTC

301 GCCACTCCACAAGCGCCCAAACCCGGCCCGGTGCAGCCGCTGACCGTGCA
CGGTGAGGTGTTCGCGGGTTTGGGCCGGGCCACGTCGGCGACTGGCACGT

351 GGGCCTCCAGCCAGTCCACGTGGCTCAAGAGGTGCAGCAGCTCCAGCAGG
CCCGGAGGTCGGTCAGGTGCACCGAGTTCTCCACGTCGTCGAGGTCGTCC

401 TGCCCGTCCCACACGTGTACTCCAGCCAGGTGCAGTATGTGGAGGGCGGC
ACGGGCAGGGTGTGCACATGAGGTCGGTCCACGTCATACACCTCCCGCCG

451 GATNCCAGCTACACNGCCAGTGCCATCCGTTCCAGCACCTACTCCTATCC
CTANGGTCGATGTGNCGGTCACGGTAGGCAAGGTCGTGGATGAGGATAGG

501 CGAGACGCCGCTGTACACGCAGACGGCAAGCACCAGCTACTACGAGGCCG
GCTCTGCGGCGACATGTGCGTCTGCCGTTCGTGGTCGATGATGCTCCGGC

551 CAGGCACGGCCACCCAGGTCAGCACCCCCGCCACCTCCCAGGCGGTGGCC
GTCCGTGCCGGTGGGTCCAGTCGTGGGGGCGGTGGAGGGTCCGCCACCGG

601 AGCAGTGGCTCCATGCCCATGTACGTGTCCGGCAGCCAGGTCGTCGCCAG
TCGTCACCGAGGTACGGGTACATGCACAGGCCGTCGGTCCAGCAGCGGTC

651 CTCCGCCAGCACTGGGGCTGGGGCCAGCAACAGCAGCGGAGGTGGTGGCA
GAGGCGGTCGTGACCCCGACCCCGGTCGTTGTCGTCGCCTCCACCACCGT

701 GTGGTGGTGGCGGCGGCGGCGGGGAGGCGGTGGCGGGGTGGCAGTGGC
CACCACCACCGCCGCCGCCGCCCCTCCGCCACCGCCCCACCGTCACCG

751 AGCACCGGAGGCGGCGGCAGCGGAGCAGGCACCTACGTGATCCAAGGCGG
TCGTGGCCTCCGCCGCCGTCGCCTCGTCCGTGGATGCACTAGGTTCCGCC

801 CTACATGCTGGGCAGTGCCAGCCAGTCTTACTCTCACACCACCCGTGCCT
GATGTACGACCCGTCACGGTCGGTCAGAATGAGAGTGTGGTGGGCACGGA

851 CGCCAGCCACGGTCCAGTGGCTCCTGGACAACTATGAGACGGCTGAGGGC
GCGGTCGGTGCCAGGTCACCGAGGACCTGTTGATACTCTGCCGACTCCCG

901 GTGAGTCTGCCACGGAGCACCCTCTACTGCCACTACTTACTGCACTGCCA
CACTCAGACGGTGCCTCGTGGGAGATGACGGTGATGAATGACGTGACGGT

951 GGAGCAGAAGCTGGAGCCCGTCAACGCCGCCTCCTTCGGCAAGCTCATCC
CCTCGTCTTCGACCTCGGGCAGTTGCGGCGGAGGAAGCCGTTCGAGTAGG

1001 GCTCCGTCTTCATGGGCCTGCGAACCCGCCGTCTGGGCACCAGGGGCAAC
CGAGGCAGAAGTACCCGGACGCTTGGGCGGCAGACCCGTGGTCCCCGTTG

1051 TCCAAGTACCACTACTATGGCCTGCGCATCAAGGCCAGCTCACCCCTGCT
AGGTTCATGGTGATGATACCGGACGCGTAGTTCCGGTCGAGTGGGGACGA

1101 GCGGCTGATGGAGGACCAGCAGCACATGGCCATGCGGGGCCAGCCCTTCT
CGCCGACTACCTCCTGGTCGTCGTGTACCGGTACGCCCCGGTCGGGAAGA

1151 CGCAGAAGCAGAGGCTCAAGCCCATCCAGAAGATGGAAGGCATGACCAAC
GCGTCTTCGTCTCCGAGTTCGGGTAGGTCTTCTACCTTCCGTACTGGTTG

1201 GGCGTGGCGGTGGGGCAGCAGCCGAGCACGGGGCTGTCGGACATCAGCGC
CCGCACCGCCACCCCGTCGTCGGCTCGTGCCCCGACAGCCTGTAGTCGCG

1251 CCAGGTGCAGCAGTACCAGCAATTTTTGGATGCCTCTCGGAGCCTCCCTG
GGTCCACGTCGTCATGGTCGTTAAAAACCTACGGAGAGCCTCGGAGGGAC

1301 ACTTCACAGAGCTCGACCTCCAGGGCAAGGTGCTGCCTGAGGGCGTCNGG
TGAAGTGTCTCGAGCTGGAGGTCCCGTTCCACGACGGACTCCCGCAGNCC

1351 CCCGGGGACATCAAAGCCTTCCAGGTCCTGTACCGGGAACACTGTGAGGC
GGGCCCCTGTAGTTTCGGAAGGTCCAGGACATGGCCCTTGTGACACTCCG

1401 CATTGTCGACNTCATGGTGAACCTGCAGTTCACCCTGGTGGAGACGCTGT
GTAACAGCTGNAGTACCACTTGGACGTCAAGTGGGACCACCTCTGCGACA

1451 GGAAGACCTTCTGGAGGTACAACCTCAGCCAGCCCAGTGAGGCGCCACCG
CCTTCTGGAAGACCTCCATGTTGGAGTCGGTCGGGTCACTCCGCGGTGGC

1501 CTGGCTGTACATGACGAGGCCGAGAAGCGACTGCCCAAAGCCATCCTGGT
GACCGACATGTACTGCTCCGGCTCTTCGCTGACGGGTTTCGGTAGGACCA

1551 GCTCCTCTCCAAGTTCGAGCCCGTGCTCCAATGGACCAAGCACTGTGACA
CGAGGAGAGGTTCAAGCTCGGGCACGAGGTTACCTGGTTCGTGACACTGT

1601 ACGTGCTGTACCAGGGCCTGGTGGAAATCCTCATTCCCGACGTGCTGCGG
TGCACGACATGGTCCCGGACCACCTTTAGGAGTAAGGGCTGCACGACGCC

1651 CCCATCCCCAGTGCCTTGACCCAAGCGATCCGGAACTTTGCCAAGAGCCT
GGGTAGGGGTCACGGAACTGGGTTCGCTAGGCCTTGAAACGGTTCTCGGA

1701 GGAGAGCTGGCTCACCCACGCCATGGTCAACATCCCCGAGGAGATGCTGC
CCTCTCGACCGAGTGGGTGCGGTACCAGTTGTAGGGGCTCCTCTACGACG

1751 GGGTGAAGGTGGCNNCGGCTGGCGCCTTCGCGCAGACACTGCGGCGCTAC
CCCACTTCCACCGNNGCCGACCGCGGAAGCGCGTCTGTGACGCCGCGATG

1801 ACGTCGCTCAACCACCTGGCGCAGGCGGCGCGCTGTGCTGCAGAACAC
TGCAGCGAGTTGGTGGACCGCGTCCGCCGCGCGACACGACGTCTTGTG

1851 CGCACAGATCAACCAGATGCTGAGCGACCTCAACCGCGTGGACTTCGCCA
GCGTGTCTAGTTGGTCTACGACTCGCTGGAGTTGGCGCACCTGAAGCGGT

1901 ACGTGCAGGAGCAGGCCTCGTGGGTGTGCCGCTGCGAGGACCGCGTGGTG
TGCACGTCCTCGTCCGGAGCACCCACACGGCGACGCTCCTGGCGCACCAC

1951 CAGCGGCTGGAGCAGGACTTCAAGGTGACGCTGCAGCAGCAGAACTCGCT
GTCGCCGACCTCGTCCTGAAGTTCCACTGCGACGTCGTCGTCTTGAGCGA

2001 GGAGCAGTGGGCGGCCTGGCTGGACGGCGTGGTGAGCCAGGTGCTCAAGC
CCTCGTCACCCGCCGGACCGACCTGCCGCACCACTCGGTCCACGAGTTCG

2051 CCTACCAGGCAGCGCGGCTTCCCCAAGGCCGCCAAGCTCTTCCTCCTCAA
GGATGGTCCGTCGCGCCGAAGGGGTTCCGGCGGTTCGAGAAGGAGGAGTT

2101 GTGGTCCTTCTACAGCTCCATGGTGATCCGGGACCTGACCCTGCGCACGG
CACCAGGAAGATGTCGAGGTACCACTAGGCCCTGGACTGGGACGCGTGCC

2151 CCGCCAGCTTCGGTTCCTTCCACCTCATCCGGCTGCTCTACGACGAGTAC
GGCGGTCGAAGCCAAGGAAGGTGGAGTAGGCCGACGAGATGCTGCTCATG

2201 ATGTACTACCTGATCGAGCACCGCGTAGCCCAGGCCAAGGGCGAGACCCC
TACATGATGGACTAGCTCGTGGCGCATCGGGTCCGGTTCCCGCTCTGGGG

2251 CATCGCCGTCATGGGCGAGTTCGCCAATCTGGCCACCTCCCTGAACCCCC
GTAGCGGCAGTACCCGCTCAAGCGGTTAGACCGGTGGAGGGACTTGGGGG

2301 TGGACCCCGACAAAGACGAGGAGGAAGAAGAGGAGGAGGAGAGCGAGGAC
ACCTGGGGCTGTTTCTGCTCCTCCTTCTTCTCCTCCTCCTCTCGCTCCTG

2351 GAGCTGCCGCAGGACATCTCACTGGCGGCTGGCGGCGAGTCACCCGCGCT
CTCGACGGCGTCCTGTAGAGTGACCGCCGACCGCCGCTCAGTGGGCGCGA

2401 GGGCCCGGAGACCCTGGAGCCGCCGGCCAAGCTGGCGCGG
CCCGGGCCTCTGGGACCTCGGCGGCCGGTTCGACCGCGCC

11. Procédé selon la revendication 9 dans lequel la séquence d'ADN code la protéine RF-X humaine entière comprenant les positions de nucléotides suivantes : 
10        20        30        40        50
|         |         |         |         |
1 GCGAAAAGCGTTTCCGCAGACAGAAGTGGGGAGAAGCGGAGGAATTAAAA
CGCTTTTCGCAAAGGCGTCTGTCTTCACCCCTCTTCGCCTCCTTAATTTT

51 AAAAAAAAAAGCCTTATTTATTATCATTTTCCCCACCGTTGGCATGGCAA
TTTTTTTTTTCGGAATAAATAATAGTAAAAGGGGTGGCAACCGTACCGTT

101 CACAGGCGTATACTGAGCTACAGGCAGCCCCGCCACCATCCCAGCCGCCA
GTGTCCGCATATGACTCGATGTCCGTCGGGGCGGTGGTAGGGTCGGCGGT

151 CAGGCCCCGCCACAAGCCCAGCCCCAGCCGCCACCGCCACCACCCCCAGC
GTCCGGGGCGGTGTTCGGGTCGGGGTCGGCGGTGGCGGTGGTGGGGGTCG

201 GGCACCCCAGCCCCCGCAGCCACCCACCGCTGCTGCCACCCCTCAGCCCC
CCGTGGGGTCGGGGGCGTCGGTGGGTGGCGACGACGGTGGGGAGTCGGGG

251 AATATGTCACCGAGCTGCAGAGCCCCCAGCCCCAGGCACAGCCACCGGGT
TTATACAGTGGCTCGACGTCTCGGGGGTCGGGGTCCGTGTCGGTGGCCCA

301 GGCCAGAAGCAGTACGTGACGGAGCTCCCGGCTGTACCCGCACCCTCGCA
CCGGTCTTCGTCATGCACTGCCTCGAGGGCCGACATGGGCGTGGGAGCGT

351 GCCAACCGGTGCACCCACCCCTTCGCCTGCACCCCAGCAGTACATCGTGG
CGGTTGGCCACGTGGGTGGGGAAGCGGACGTGGGGTCGTCATGTAGCACC

401 TCACTGTCTCTGAAGGTGCCATGCGGGCCAGCGAGACAGTGTCGGAGGCC
AGTGACAGAGACTTCCACGGTACGCCCGGTCGCTCTGTCACAGCCTCCGG

451 AGCCCCGGCTCCACCGCCAGCCAGACCGGCGTTCCTACTCAGGTGGTTCA
TCGGGGCCGAGGTGGCGGTCGGTCTGGCCGCAAGGATGAGTCCACCAAGT

501 GCAGGTGCAGGGCACCCAGCAGCGGCTGCTGGTCCAGACGAGCGTGCAGG
CGTCCACGTCCCGTGGGTCGTCGCCGACGACCAGGTCTGCTCGCACGTCC

551 CCAAGCCAGGCCACGTGTCGCCCCTCCAGCTGACCAACATCCAAGTGCCC
GGTTCGGTCCGGTGCACAGCGGGGAGGTCGACTGGTTGTAGGTTCACGGG

601 CAGCAGGCTCTTCCCACGCAGCGTCTGGTGGTGCAGAGCGCAGCCCCAGG
GTCGTCCGAGAAGGGTGCGTCGCAGACCACCACGTCTCGCGTCGGGGTCC
 
651 CAGCAAAGGTGGCCAGGTCTCCCTGACGGTCCATGGTACCCAGCAGGTGC
GTCGTTTCCACCGGTCCAGAGGGACTGCCAGGTACCATGGGTCGTCCACG

701 ACTCGCCCCCAGAGCAGTCGCCGGTGCAGGCCAACAGCTCTTCCAGCAAG
TGAGCGGGGGTCTCGTCAGCGGCCACGTCCGGTTGTCGAGAAGGTCGTTC

751 ACAGCCGGGGCCCCCACGGGCACAGTGCCACAGCAGCTGCAGGTCCACGG
TGTCGGCCCCGGGGGTGCCCGTGTCACGGTGTCGTCGACGTCCAGGTGCC

801 CGTCCAGCAGAGTGTCCCCGTCACCCAAGAGAGATCTGTGGTCCAGGCCA
GCAGGTCGTCTCACAGGGGCAGTGGGTTCTCTCTAGACACCAGGTCCGGT

851 CTCCACAAGCGCCCAAACCCGGCCCGGTGCAGCCGCTGACCGTGCAGGGC
GAGGTGTTCGCGGGTTTGGGCCGGGCCACGTCGGCGACTGGCACGTCCCG

901 CTCCAGCCAGTCCACGTGGCTCAAGAGGTGCAGCAGCTCCAGCAGGTGCC
GAGGTCGGTCAGGTGCACCGAGTTCTCCACGTCGTCGAGGTCGTCCACGG

951 CGTCCCACACGTGTACTCCAGCCAGGTGCAGTATGTGGAGGGCGGCGATG
GCAGGGTGTGCACATGAGGTCGGTCCACGTCATACACCTCCCGCCGCTAC

1001 CCAGCTACACGGCCAGTGCCATCCGTTCCAGCACCTACTCCTATCCCGAG
GGTCGATGTGCCGGTCACGGTAGGCAAGGTCGTGGATGAGGATAGGGCTC

1051 ACGCCGCTGTACACGCAGACGGCAAGCACCAGCTACTACGAGGCCGCAGG
TGCGGCGACATGTGCGTCTGCCGTTCGTGGTCGATGATGCTCCGGCGTCC

1101 CACGGCCACCCAGGTCAGCACCCCCGCCACCTCCCAGGCGGTGGCCAGCA
GTGCCGGTGGGTCCAGTCGTGGGGGCGGTGGAGGGTCCGCCACCGGTCGT

1151 GTGGCTCCATGCCCATGTACGTGTCCGGCAGCCAGGTCGTCGCCAGCTCC
CACCGAGGTACGGGTACATGCACAGGCCGTCGGTCCAGCAGCGGTCGAGG

1201 GCCAGCACTGGGGCTGGGGCCAGCAACAGCAGCGGAGGTGGTGGCAGTGG
CGGTCGTGACCCCGACCCCGGTCGTTGTCGTCGCCTCCACCACCGTCACC

1251 TGGTGGCGGCGGCGGCGGGGGAGGCGGTGGCGGGGGTGGCAGTGGCAGCA
ACCACCGCCGCCGCCGCCCCCTCCGCCACCGCCCCCACCGTCACCGTCGT
 
1301 CCGGAGGCGGCGGCAGCGGAGCAGGCACCTACGTGATCCAAGGCGGCTAC
GGCCTCCGCCGCCGTCGCCTCGTCCGTGGATGCACTAGGTTCCGCCGATG

1351 ATGCTGGGCAGTGCCAGCCAGTCTTACTCTCACACCACCCGTGCCTCGCC
TACGACCCGTCACGGTCGGTCAGAATGAGAGTGTGGTGGGCACGGAGCGG

1401 AGCCACGGTCCAGTGGCTCCTGGACAACTATGAGACGGCTGAGGGCGTGA
TCGGTGCCAGGTCACCGAGGACCTGTTGATACTCTGCCGACTCCCGCACT

1451 GTCTGCCACGGAGCACCCTCTACTGCCACTACTTACTGCACTGCCAGGAG
CAGACGGTGCCTCGTGGGAGATGACGGTGATGAATGACGTGACGGTCCTC

1501 CAGAAGCTGGAGCCCGTCAACGCCGCCTCCTTCGGCAAGCTCATCCGCTC
GTCTTCGACCTCGGGCAGTTGCGGCGGAGGAAGCCGTTCGAGTAGGCGAG

1551 CGTCTTCATGGGCCTGCGAACCCGCCGTCTGGGCACCAGGGGCAACTCCA
GCAGAAGTACCCGGACGCTTGGGCGGCAGACCCGTGGTCCCCGTTGAGGT

1601 AGTACCACTACTATGGCCTGCGCATCAAGGCCAGCTCACCCCTGCTGCGG
TCATGGTGATGATACCGGACGCGTAGTTCCGGTCGAGTGGGGACGACGCC

1651 CTGATGGAGGACCAGCAGCACATGGCCATGCGGGGCCAGCCCTTCTCGCA
GACTACCTCCTGGTCGTCGTGTACCGGTACGCCCCGGTCGGGAAGAGCGT

1701 GAAGCAGAGGCTCAAGCCCATCCAGAAGATGGAAGGCATGACCAACGGCG
CTTCGTCTCCGAGTTCGGGTAGGTCTTCTACCTTCCGTACTGGTTGCCGC

1751 TGGCGGTGGGGCAGCAGCCGAGCACGGGGCTGTCGGACATCAGCGCCCAG
ACCGCCACCCCGTCGTCGGCTCGTGCCCCGACAGCCTGTAGTCGCGGGTC

1301 GTGCAGCAGTACCAGCAATTTTTGGATGCCTCTCGGAGCCTCCCTGACTT
CACGTCGTCATGGTCGTTAAAAACCTACGGAGAGCCTCGGAGGGACTGAA

1851 CACAGAGCTCGACCTCCAGGGCAAGGTGCTGCCTGAGGGCGTCGGCCCG
GTGTCTCGAGCTGGAGGTCCCGTTCCACGACGGACTCCCGCAGCCGGGC

1901 GGGACATCAAAGCCTTCCAGGTCCTGTACCGGGAACACTGTGAGGCCATT
CCCTGTAGTTTCGGAAGGTCCAGGACATGGCCCTTGTGACACTCCGGTAA

1951 GTCGACGTCATGGTGAACCTGCAGTTCACCCTGGTGGAGACGCTGTGGAA
CAGCTGCAGTACCACTTGGACGTCAAGTGGGACCACCTCTGCGACACCTT
 
2001 GACCTTCTGGAGGTACAACCTCAGCCAGCCCAGTGAGGCGCCACCGCTGG
CTGGAAGACCTCCATGTTGGAGTCGGTCGGGTCACTCCGCGGTGGCGACC

2051 CTGTACATGACGAGGCCGAGAAGCGACTGCCCAAAGCCATCCTGGTGCTC
GACATGTACTGCTCCGGCTCTTCGCTGACGGGTTTCGGTAGGACCACGAG

2101 CTCTCCAAGTTCGAGCCCGTGCTCCAATGGACCAAGCACTGTGACAACGT
GAGAGGTTCAAGCTCGGGCACGAGGTTACCTGGTTCGTGACACTGTTGCA

2151 GCTGTACCAGGGCCTGGTGGAAATCCTCATTCCCGACGTGCTGCGGCCCA
CGACATGGTCCCGGACCACCTTTAGGAGTAAGGGCTGCACGACGCCGGGT

2201 TCCCCAGTGCCTTGACCCAAGCGATCCGGAACTTTGCCAAGAGCCTGGAG
AGGGGTCACGGAACTGGGTTCGCTAGGCCTTGAAACGGTTCTCGGACCTC

2251 AGCTGGCTCACCCACGCCATGGTCAACATCCCCGAGGAGATGCTGCGGGT
TCGACCGAGTGGGTGCGGTACCAGTTGTAGGGGCTCCTCTACGACGCCCA

2301 GAAGGTGGCCGCGGCTGGCGCCTTCGCGCAGACACTGCGGCGCTACACGT
CTTCCACCGGCGCCGACCGCGGAAGCGCGTCTGTGACGCCGCGATGTGCA

2351 CGCTCAACCACCTGGCGCAGGCGGCGCGCGCTGTGCTGCAGAACACCGCA
GCGAGTTGGTGGACCGCGTCCGCCGCGCGCGACACGACGTCTTGTGGCGT

2401 CAGATCAACCAGATGCTGAGCGACCTCAACCGCGTGGACTTCGCCAACGT
GTCTAGTTGGTCTACGACTCGCTGGAGTTGGCGCACCTGAAGCGGTTGCA

2451 GCAGGAGCAGGCCTCGTGGGTGTGCCGCTGCGAGGACCGCGTGGTGCAGC
CGTCCTCGTCCGGAGCACCCACACGGCGACGCTCCTGGCGCACCACGTCG

2501 GGCTGGAGCAGGACTTCAAGGTGACGCTGCAGCAGCAGAACTCGCTGGAG
CCGACCTCGTCCTGAAGTTCCACTGCGACGTCGTCGTCTTGAGCGACCTC

2551 CAGTGGGCGGCCTGGCTGGACGGCGTGGTGAGCCAGGTGCTCAAGCCCTA
GTCACCCGCCGGACCGACCTGCCGCACCACTCGGTCCACGAGTTCGGGAT

2601 CCAGGGCAGCGCCGGCTTCCCCAAGGCCGCCAAGCTCTTCCTCCTCAAGT
GGTCCCGTCGCGGCCGAAGGGGTTCCGGCGGTTCGAGAAGGAGGAGTTCA

2651 GGTCCTTCTACAGCTCCATGGTGATCCGGGACCTGACCCTGCGCAGCGCC
CCAGGAAGATGTCGAGGTACCACTAGGCCCTGGACTGGGACGCGTCGCGG
 
2701 GCCAGCTTCGGTTCCTTCCACCTCATCCGGCTGCTCTACGACGAGTACAT
     CGGTCGAAGCCAAGGAAGGTGGAGTAGGCCGACGAGATGCTGCTCATGTA

2751 GTACTACCTGATCGAGCACCGCGTAGCCCAGGCCAAGGGCGAGACCCCCA
     CATGATGGACTAGCTCGTGGCGCATCGGGTCCGGTTCCCGCTCTGGGGGT

2801 TCGCCGTCATGGGCGAGTTCGCCAATCTGGCCACCTCCCTGAACCCCCTG
     AGCGGCAGTACCCGCTCAAGCGGTTAGACCGGTGGAGGGACTTGGGGGAC

2851 GACCCCGACAAAGACGAGGAGGAAGAAGAGGAGGAGAGCGAGGACGA
     CTGGGGCTGTTTCTGCTCCTCCTTCTTCTCCTCCTCTCGCTCCTGCT

2901 GCTGCCGCAGGACATCTCACTGGCGGCTGGCGGCGAGTCACCCGCGCTGG
     CGACGGCGTCCTGTAGAGTGACCGCCGACCGCCGCTCAGTGGGCGCGACC

2951 GCCCGGAGACCCTGGAGCCGCCGGCCAAGCTGGCGCGGACTGACGCGCGC
     CGGGCCTCTGGGACCTCGGCGGCCGGTTCGACCGCGCCTGACTGCGCGCG

3001 GGCCTCTTCGTGCAGGCGCTGCCCTCCAGCTAAGCCCTTGGCCTCCCCGC
     CCGGAGAAGCACGTCCGCGACGGGAGGTCGATTCGGGAACCGGAGGGGCG

3051 CCCACCCGCCCCCGCCACCCCTCCACGCCAGGGTCC
     GGGTGGGCGGGGGCGGTGGGGAGGTGCGGTCCCAGG
 où la région codante pour ladite protéine RF-X humaine commence en position 94 et se termine en position 3030.

12. Procédé de préparation d'une séquence d'ADN qui code une protéine selon l'une quelconque des revendications 3 à 8 et qui s'hybride avec la séquence d'ADN de la revendication 10 ou 11 dans des conditions conventionnelles, ledit procédé comprenant l'isolement de ladite séquence d'ADN à partir d'une banque d'ADNc de bactériophages recombinés exprimant un ADN codant lesdites protéines par criblage de ladite banque avec des sondes appropriées.

13. Procédé de préparation d'une séquence nucléotidique qui est complémentaire du brin codant d'une séquence d'ADN selon l'une quelconque des revendications 9 à 12, ledit procédé comprenant la synthèse de ladite séquene par des moyens enzymatiques utilisant les séquences d'ADN desdites revendications comme matrice ou par synthèse chimique.

14. Procédé de préparation d'un fragment de la séquence nucléotidique selon la revendication 12, ledit procédé comprenant la digestion enzymatique de ladite séquence nucléotidique et l'isolement du fragment souhaité.

15. Procédé selon la revendication 14 dans lequel ledit fragment est la région codante de la séquence d'ADN de la revendication 11 ou en est dérivé.

16. Procédé de préparation d'une molécule d'ADN recombiné contenant une séquence d'ADN ou une séquence nucléotidique selon l'une quelconque des revendications 9 à 15, comprenant l'insertion de ladite séquence d'ADN ou de ladite séquence nucléotidique dans un vecteur approprié.

17. Procédé selon la revendication 16 dans lequel ladite molécule d'ADN recombiné est pUC/λ9 ((DSM 5303) ou pRF-XC (DSM 5876).

18. Procédé de préparation de la molécule d'ADN recombiné selon la revendication 16 qui est le vecteur d'expression pT7/λ9, ledit procédé comprenant le sous-clonage de l'insert EcoRI de 3,7 kb de pUC/λ9 en phase dans le vecteur d'expression pT7/7.

19. Organisme hôte transformé avec une molécule d'ADN recombiné selon l'une quelconque des revendications 16 à 18.

20. Procédé de préparation d'une composition pharmaceutique contenant une protéine préparée selon l'une quelconque des revendications 1 à 8, lequel procédé comprend la combinaison de ladite protéine avec un support pharmaceutiquement acceptable.

21. Procédé de préparation d'une composition pharmaceutique contenant une séquence nucléotidique préparée selon la revendication 13 ou un fragment de celle-ci préparé selon la revendication 14 ou 15, lequel procédé comprend la combinaison de ladite séquence nucléotidique ou dudit fragment avec un support pharmaceutiquement acceptable.

22. Procédé de préparation d'une composition pharmaceutique contenant une protéine préparée selon l'une quelconque des revendications 1 à 6 ou 8 pour le traitement des maladies dans lesquelles une augmentation du niveau de l'expression des gènes CMH de classe II est souhaitable, lequel procédé comprend la combinaison de ladite protéine avec un support pharmaceutiquement acceptable.

23. Procédé de préparation d'une composition pharmaceutique contenant une protéine préparée selon la revendication 7 ou une protéine recombinée correspondante préparée selon la revendication 8 pour le traitement des maladies dans lesquelles une diminution du niveau de l'expression des gènes CMH de classe II est souhaitable, lequel procédé comprend la combinaison de ladite protéine ou dudit de ladite protéine recombinée avec un support pharmaceutiquement acceptable.

24. Procédé de préparation d'une composition pharmaceutique contenant une séquence nucléotidique préparée selon la revendication 13 ou un fragment de celle-ci préparé selon la revendication 14 ou 15 pour le traitement des maladies dans lesquelles une diminution du niveau de l'expression des gènes CMH de classe II est souhaitable, lequel procédé comprend la combinaison de ladite séquence nucléotidique ou dudit fragment avec un support pharmaceutiquement acceptable.

25. Utilisation d'une protéine préparée selon l'une quelconque des revendications 1 à 8 pour le criblage et l'identification de substances capables d'inhiber l'expression des gènes CMH de classe II.

26. Procédé de préparation d'une composition pharmaceutique contenant une protéine préparée selon l'une quelconque des revendications 1 à 8 ou une séquence nucléotidique préparée selon la revendication 13 ou un fragment de celle-ci préparé selon la revendication 14 ou 15 ou une substance qui inhibe l'expression des gènes CMH de classe II identifiée selon la revendication 25 pour le traitement des troubles immuns, lequel procédé comprend la combinaison de ladite protéine, de ladite séquence nucléotidique dudit fragment, ou de ladite substance avec un support pharmaceutiquement acceptable.
